# EUROPEAN PATENT SPECIFICATION

(11) **EP 4 274 561 B1**
(45) Date of publication and mention of the grant of the patent: **02.07.2025**
(21) Application number: 22736617.6
(22) Date of filing: 10.01.2022
(51) Int. Cl.: A61P 35/00, A61K 9/00, A61K 31/185, A61K 47/40, A61K 47/69, C08B 37/16

(54) **PHARMACEUTICAL COMPOSITIONS OF CHEMOTHERAPEUTIC AGENTS BASED ON BETA-SUBSTITUTED BETA-AMINO ACID DERIVATIVES**
PHARMAZEUTISCHE ZUSAMMENSETZUNGEN VON CHEMOTHERAPEUTISCHEN MITTELN AUF BASIS VON BETA-SUBSTITUIERTEN BETA-AMINOSÄUREDERIVATEN
COMPOSITIONS PHARMACEUTIQUES D'AGENTS CHIMIOTHÉRAPEUTIQUES À BASE DE DÉRIVÉS D'ACIDES BÊTA-AMINÉS BÊTA-SUBSTITUÉS

(30) Priority: 08.01.2021 WO PCT/CN2021/070782
(43) Date of publication of application: 15.11.2023
(73) Proprietor: Quadriga Biosciences, Inc., Los Altos, California 94022 (US)
(72) Inventor: JANDELEIT, Bernd, Menlo Park California 94025 (US); TAM, Peter, Redwood City California 94061 (US); LIU, Ruifang, Shanghai 201210 (CN); YAN, Shixiang, Shanghai 201210 (CN)
(74) Representative: Marks & Clerk LLP
(86) International application number: PCT/CN2022/070958
(87) International publication number: WO 2022/148456

(56) References cited:
- WO-A1-2010/138920
- WO-A1-2015/117147
- WO-A1-2017/024009
- AU-A1- 2011 204 957
- AU-A1- 2013 201 437
- US-A1- 2015 218 086
- US-A1- 2016 346 240
- DAS O., ET AL. : "Utility of Sulfobutyl Ether beta-Cyclodextrin Inclusion Complexes in Drug Delivery: A Review", INDIAN JOURNAL OF PHARMACEUTICAL SCIENCES, vol. 81, no. 4, 31 July 2019 (2019-07-31), pages 589 - 600, XP055949117
- KIM YESOOK, ET AL.: "Inclusion complexation of ziprasidone mesylate with β-cyclodextrin sulfobutyl ether.", JOURNAL OF PHARMACEUTICAL SCIENCES, vol. 87, no. 12, 2 October 1998 (1998-10-02), pages 1560 - 1567, XP055949124, DOI: 10.1021/js980109t

## Description

This application claims the benefit of PCT International Application No. PCT/CN2021/070782 filed on January 8, 2021.

### FIELD

The disclosure relates to pharmaceutical compositions of chemotherapeutic agents based on β-substituted β-amino acid derivatives. The pharmaceutical compositions include a β-substituted β-amino acid derivative and a cyclodextrin derivative. The pharmaceutical compositions are useful for treating cancer.

### BACKGROUND

The ability to selectively target chemotherapy has immense value in clinical practice. Cancer is a leading cause of death in the developed world, with one in every three people developing cancer during his or her lifetime. There are many treatment options for cancer including surgery, chemotherapy, radiation therapy, immunotherapy, and monoclonal antibody treatment. Unfortunately, for many patients, cancer treatment options are limited, and response rates remain low.

β-Substituted β-amino acid derivatives can be used as LAT1-transported chemotherapeutic agents. Certain β-substituted β-amino acid derivatives are unstable in aqueous buffered solutions suitable for intravenous administration. US2015/218086A1 discloses β-Substituted β-amino acids, β-Substituted β-amino acid derivatives, and β-Substituted β-amino acid analogs and (bio)isosteres and their use as chemotherapeutic agents are disclosed. WO2010138920A1 is directed to pharmaceutical compositions comprising melphalan and a cyclodextrin derivative, and methods of making and using the same.

### SUMMARY

According to the present invention, a guest-host inclusion complex comprises:
a mass ratio of (S)-3-amino-4-(5-(bis-chloroethyl)amino)-2-methylphenyl) butanoic acid, or a pharmaceutically acceptable zwitterion, internal salt, or salt thereof (1c), to a sulfobutyl ether-β-cyclodextrin having an average degree of substitution (ADS) of from 6 to 8 (SBE₆₋₈-β-CD) or a pharmaceutically acceptable zwitterion, internal salt, or salt thereof, of from 1:50 to 1:60.

According to the present invention, a pharmaceutical composition comprises:
a mass ratio of (S)-3-amino-4-(5-(bis-chloroethyl)amino)-2-methylphenyl) butanoic acid (1c), or a pharmaceutically acceptable zwitterion, internal salt, or salt thereof, to a sulfobutyl ether-β-cyclodextrin having an average degree of substitution (ADS) of from 6 to 8 (SBE₆₋₈-β-CD) or a pharmaceutically acceptable zwitterion, internal salt, or salt thereof, of from 1:50 to 1:60.

According to the present invention, a pharmaceutical kit comprises a guest-host inclusion complex according to the present invention, wherein the guest-host inclusion complex is a lyophilizate; and a sodium chloride solution for reconstituting the lyophilizate to provide an aqueous formulation suitable for intravenous administration.

According to the present invention, a guest-host inclusion complex, a pharmaceutical composition and a pharmaceutical kit are provided for use in methods of treating a brain cancer or metastatic cancer in the brain.

### DETAILED DESCRIPTION

For purposes of the following detailed description, it is to be understood that embodiments provided by the present disclosure may assume various alternative variations and step sequences, except where expressly specified to the contrary. Moreover, other than in any operating examples, or where otherwise indicated, all numbers expressing, for example, quantities of ingredients used in the specification and claims are to be understood as being modified in all instances by the term "about." Accordingly, unless indicated to the contrary, the numerical parameters set forth in the following specification and attached claims are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variation found in their respective testing measurements.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

"Alkoxy" refers to a radical -OR where R is alkyl. Examples of alkoxy groups include methoxy, ethoxy, propoxy, and butoxy. An alkoxy group can be, for example, C₁₋₆ alkoxy, C₁₋₅ alkoxy, C₁₋₄ alkoxy, C₁₋₃ alkoxy, ethoxy or methoxy.

"Alkyl" refers to a saturated, branched, or straight-chain, monovalent hydrocarbon radical derived by the removal of one hydrogen atom from a single carbon atom of a parent alkane. An alkyl group can be, for example, C₁₋₆ alkyl, C₁₋₅ alkyl, C₁₋₄ alkyl, or C₁₋₃ alkyl. An alkyl group can be methyl, ethyl, n-propyl, iso-propyl, or *tert-butyl.*

"Alkanediyl" refers to a saturated, branched, or straight-chain, divalent hydrocarbon radical derived by the removal of two hydrogen atoms from one or two carbon atoms of a parent alkane. An alkanediyl can be, for example, C₁₋₈ alkanediyl, C₁₋₆ alkanediyl, C₁₋₅ alkanediyl, C₁₋₄ alkanediyl, or C₁₋₃ alkanediyl. An alkanediyl can be, for example, methane-diyl, ethane-diyl, n-propane-diyl, iso-propane-diyl, or butane-diyl.

"Alkanediyl sulfonate salt" refers to an alkanediyl group in which one of carbon atoms is bonded to a sulfonate salt, -SO₃⁻X⁺, group, where X⁺ is a counter cation. In an alkanediyl sulfonate salt the sulfonate salt can be associated with the terminal carbon of the alkanediyl group. An alkanediyl sulfonate salt can be, for example, C₁₋₈ alkanediyl sulfonate salt, C₁₋₆ alkanediyl sulfonate salt, a C₁₋₅ alkanediyl sulfonate salt, a C₁₋₄ alkanediyl sulfonate salt, or a C₁₋₃ alkanediyl sulfonate salt. In an alkanediyl sulfonate salt the terminal carbon atom can be substituted with the sulfonate salt. For example, a C₁₋₄ alkanediyl sulfonate salt can have the structure -CH₂-SO₃⁻X⁺, -CH₂-CH₂-SO₃⁻X⁺, or -CH₂-CH₂-CH₂-SOₐ⁻X⁺, or -CH₂-CH₂-CH₂-CH₂-SOₐ⁻X⁺. The counter cation can be, for example, Na⁺ and a C₁₋₈ alkanediyl sulfonate.

"Substituted" refers to a group in which one or more hydrogen atoms are independently replaced with the same or different substituent(s). Each substituent can be independently selected from halogen, - OH, -CN, -CF₃, -OCF₃, =O (oxo), -NO₂, C₁₋₆ alkoxy, C₁₋₆ alkyl, -COOR, -NR₂, and -CONR₂; wherein each R is independently selected from hydrogen and C₁₋₆ alkyl. Each substituent can be independently selected from halogen, -NH₂, -OH, C₁₋₃ alkoxy, and C₁₋₃ alkyl, trifluoromethoxy, and trifluoromethyl. Each substituent can be independently selected from -OH, methyl, ethyl, trifluoromethyl, methoxy, ethoxy, and trifluoromethoxy. Each substituent can be selected from C₁₋₃ alkyl, =O (oxo), C₁₋₃ alkyl, C₁₋₃ alkoxy, and phenyl. Each substituent can be selected from -OH, -NH₂, C₁₋₃ alkyl, and C₁₋₃ alkoxy.

"Average degree of substitution" (ADS) refers to the average number of substituent groups per cyclodextrin molecule. The concept of an average degree of substitution for a cyclodextrin derivative is described in PCT International Publication No. WO 2009/018069. As an example, the following notation can be used to describe a cyclodextrin derivative. The substituent(s) are abbreviated with a subscript denoting the ADS of the substituents. For example, a sulfobutyl ether-derivatized β-cyclodextrin having an ADS of 6.5 is denoted as SBE_{6.5}-β-CD, where SBE is an abbreviation for a sulfobutyl ether group. As another example, a β-cyclodextrin derivatized with both sulfobutyl ether and hydroxypropyl groups is denoted as SBE_{4.2}-HP_{2.5}-β-CD where the ADS of the sulfobutyl ether groups (SBE) is 4.2 and the ADS of the hydroxypropyl (HP) groups is 2.5.

"Compounds" disclosed herein include any specific compounds within the disclosed formula. Compounds may be identified either by their chemical structure and/or chemical name. Compounds are named using the ChemBioDraw Ultra 14.0.0.117 (CambridgeSoft, Cambridge, MA) nomenclature program. When the chemical structure and chemical name conflict, the chemical structure is determinative of the identity of the compound. The compounds described herein may comprise one or more stereogenic centers and/or double bonds and therefore may exist as stereoisomers such as double-bond isomers (*i.e.*, geometric isomers), enantiomers, diastereomers, tautomers, or atropisomers. Accordingly, any chemical structures within the scope of the specification depicted, in whole or in part, with a relative configuration encompass all possible enantiomers and stereoisomers of the illustrated compounds including the stereoisomerically pure form (*e.g*., geometrically pure, enantiomerically pure, or diastereomerically pure) and enantiomeric and (dia)stereoisomeric mixtures. Enantiomeric and (dia)stereoisomeric mixtures may be resolved into their component enantiomers or (dia)stereoisomers using separation techniques or chiral synthesis techniques well known to a person skilled in the art.

A compound of Formula (1) encompasses a compound of Formula (1a), a compound of Formula (1b), a compound of Formula (1c), and a combination of any of the foregoing.

"Cyclodextrin derivative" refers to a cyclic oligosaccharide comprising five or more α-D-glucopyranoside units linked in a circular configuration and comprising a substituent group bonded to one or more of the glucopyranoside units at the 2, 3, and/or 6 position(s) through a γ-1,4-glycosidic bond.

A "nominal concentration" of a compound of Formula (1) refers to the concentration of the complexed compound of Formula (1) and the un-complexed compound of Formula (1) in a composition or solution.

"Patient" refers to a mammal, for example, a human.

"Pharmaceutically acceptable" refers to approved or approvable by a regulatory agency of the Federal or a state government or listed in the U.S. Pharmacopoeia or other generally recognized pharmacopoeia for use in animals, and more particularly in humans.

"Pharmaceutically acceptable salt" refers to a salt of a compound, which possesses the desired pharmacological activity of the parent compound. Such salts include acid addition salts, formed with inorganic acids and one or more protonable functional groups such as primary, secondary, or tertiary amines within the parent compound. Examples of suitable inorganic acids include hydrochloric acid, hydrobromic acid, sulfuric acid, nitric acid, phosphoric acid, and the like. A salt can be formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like. A salt can be formed when one or more acidic protons present in the parent compound are replaced by a metal ion, *e.g.*, an alkali metal ion, an alkaline earth ion, or an aluminum ion, or combinations thereof; or coordinates with an organic base such as ethanolamine, diethanolamine, triethanolamine, N-methylglucamine, and the like. A pharmaceutically acceptable salt can be the hydrochloride salt. A pharmaceutically acceptable salt can be the sodium salt. In compounds having two or more ionizable groups, a pharmaceutically acceptable salt can comprise one or more counterions, such as a bi-salt, for example, a dihydrochloride salt. Examples of pharmaceutically acceptable salts are disclosed, for example, in Stahl and Wermuth (Eds), Handbook of Pharmaceutical Salts, Properties, Selection and Use, First Edition, Wiley-VCH, 2008.

"Pharmaceutically acceptable salt" includes hydrates and other solvates, as well as salts in crystalline or non-crystalline form. Where a particular pharmaceutically acceptable salt is disclosed, it is understood that the particular salt (*e.g.*, a hydrochloride salt) is an example of a salt, and that other salts may be formed using techniques known to one of skill in the art. Additionally, one of skill in the art would be able to convert the pharmaceutically acceptable salt to the corresponding compound, free base and/or free acid, using techniques generally known in the art.

"Pharmaceutically acceptable vehicle" refers to a pharmaceutically acceptable diluent, a pharmaceutically acceptable adjuvant, a pharmaceutically acceptable excipient, a pharmaceutically acceptable carrier, or a combination of any of the foregoing with which a compound provided by the present disclosure may be administered to a patient and which does not destroy the pharmacological activity thereof and which is non-toxic when administered in doses sufficient to provide a therapeutically effective amount of the compound.

"Pharmaceutical composition" refers to a β-substituted β-amino acid derivative or a pharmaceutically acceptable salt thereof and at least one pharmaceutically acceptable vehicle, with which the β-substituted β-amino acid derivative or a pharmaceutically acceptable salt thereof is administered to a patient. Pharmaceutically acceptable vehicles are known in the art.

"Curing" a disease refers to eliminating a disease or disorder or eliminating a symptom of a disease or disorder.

"Disease" refers to a disease, disorder, condition, or symptom of any of the foregoing.

"Treating" or "treatment" of a disease or disorder refers to reducing the severity of one or more clinical symptom of the disease or disorder, delaying the onset of one or more clinical symptoms of the disease or disorder, and/or mitigating one or more clinical symptoms of the disease or disorder.

"Treating" or "treatment" of a disease or disorder refers to inhibiting the disease or disorder or one or more clinical symptoms of the disease or disorder, arresting the development of the disease or disorder or one or more clinical symptoms of the disease or disorder, relieving the disease or disorder or one or more clinical symptoms of the disease or disorder, causing the regression of the disease or disorder or one or more clinical symptoms of the disease or disorder, and/or stabilization of the disease or disorder or one or more clinical symptoms of the disease or disorder, "Treating" or "treatment" of a disease or disorder refers to producing a clinically beneficial effect without curing the underlying disease or disorder.

"Therapeutically effective amount" refers to the amount of a compound such as pharmaceutically active ingredient that, when administered to a patient for treating a disease, or at least one of the clinical symptoms of a disease, is sufficient to affect such treatment of the disease or symptom thereof. A "therapeutically effective amount" may vary depending, for example, on the compound, the disease and/or symptoms of the disease, the severity of the disease and/or symptoms of the disease or disorder, the age, weight, and/or health of the patient to be treated, and the judgment of the prescribing physician. A therapeutically effective amount in any given instance may be ascertained by those skilled in the art or capable of determination by routine experimentation.

"Therapeutically effective dose" refers to a dose that provides effective treatment of a disease or disorder in a patient. A therapeutically effective dose may vary from compound to compound, and from patient to patient, and may depend upon factors such as the condition of the patient and the route of delivery. A therapeutically effective dose may be determined in accordance with routine pharmacological procedures known to those skilled in the art.

"Vehicle" refers to a diluent, excipient or carrier with which a compound is administered to a patient. A vehicle can be a pharmaceutically acceptable vehicle. Pharmaceutically acceptable vehicles are known in the art.

"About" refers to within 5% of a specific value, for example, within 5% of a stated concentration range or within 5% of a stated time frame.

Reference is now made to pharmaceutical compositions and methods of using the pharmaceutical compositions. The disclosed pharmaceutical compositions and methods of using the pharmaceutical compositions are not intended to be limiting of the claims. To the contrary, the claims are intended to cover all alternatives, modifications, and equivalents.

A guest-host inclusion complex provided by the present disclosure can comprise a β-substituted β-amino acid derivative and a cyclodextrin derivative. A guest-host inclusion complex can be in the form of a lyophilizate.

A pharmaceutical composition provided by the present disclosure can comprise a β-substituted β-amino acid derivative and a cyclodextrin derivative. A pharmaceutical composition can comprise a guest-host inclusion complex provided by the present disclosure reconstituted in an aqueous formulation. A pharmaceutical composition provided by the present disclosure can be an aqueous solution for intravenous injection. A pharmaceutical composition provided by the present disclosure can be useful in treating cancer.

Cyclodextrins have been used to improve the solubility and stability of chemotherapeutic agents such as melphalan. Cyclodextrins are a family of cyclic oligosaccharides consisting of a macrocyclic ring of glucose subunits joined by α-1,4 glycosidic bonds. Cyclodextrins are produced from starch by enzymatic conversion. They are used in food, pharmaceutical, drug delivery, and chemical industries, as well as agriculture and environmental engineering. Cyclodextrins include 5 or more α-D-glucopyranoside units, as in amylose, a fragment of starch. Typical cyclodextrins contain a number of glucose monomers ranging from six to eight units in a ring, creating a toroidal shape. For example, α-cyclodextrin contains 6 glucose subunits, β-cyclodextrin contains 7 glucose subunits, and γ-cyclodextrin contains 8 glucose subunits.

Sulfoalkyl-substituted cyclodextrins are water soluble and are characterized by a hydrophilic outer surface surrounding an internal lipophilic cavity. Cyclodextrins and lipophilic therapeutic agents can form guest-host inclusion complexes that can enhance the physicochemical properties of a drug.

A guest-host inclusion complex or a pharmaceutical composition provided by the present disclosure can comprise a β-substituted β-amino acid derivative or a combination of β-substituted β-amino acid derivatives.

A β-substituted β-amino acid derivative can have the structure of Formula (1) or a combination of β-substituted β-amino acid derivatives of Formula (1): 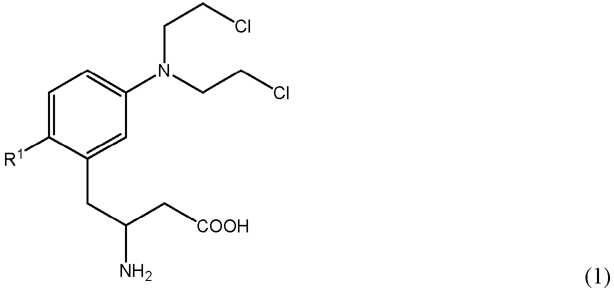 or a pharmaceutically acceptable zwitterion, internal salt, or salt thereof, wherein R¹ is selected from C₁₋₆ alkyl and C₁₋₆ alkoxy.

In a compound of Formula (1), R¹ can be C₁₋₆ alkyl.

In a compound of Formula (1), R¹ can be selected from methyl, ethyl, n-propyl, isopropyl, n-butyl, iso-butyl, sec-butyl, and *tert-*butyl*.*

In a compound of Formula (1), R¹ can be C₁₋₆ alkoxy.

In a compound of Formula (1), R¹ can be selected from methoxy, ethoxy, n-propoxy, isopropoxy, n-butoxy, iso-butoxy, sec-butoxy, and *tert*-butoxy.

In a compound of Formula (1), the carbon atom to which the amino group is bonded can be in the (S) absolute configuration.

In a compound of Formula (1), the carbon atom to which the amino group is bonded can be in the (R) absolute configuration.

A compound of Formula (1) can be a mixture having both (*S*) and (*R*) enantiomers such as a racemic mixture having a 1:1 ratio of the (*S*) and (*R*) enantiomers or a non-racemic mixture such as a mixture having about 75% of the (*S*) enantiomer and about 25% of the (*R*) enantiomer. A compound of Formula (1) can comprise, for example, X% of the (*S*) enantiomer and 100%-X% of the (*R*) enantiomer, where X is from 0 to 100.

A compound of Formula (1) can be 3-amino-4-(5-(bis(2-chloroethyl)amino)-2-methylphenyl)butanoic acid (1a), or a pharmaceutically acceptable zwitterion, internal salt, or salt thereof:

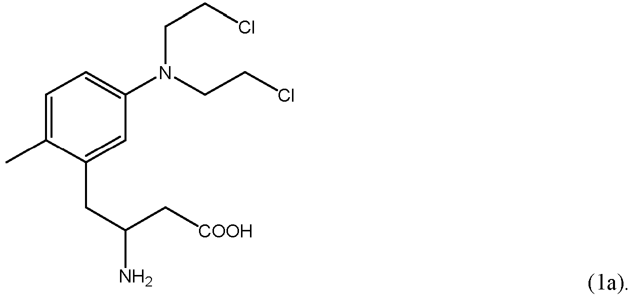

A compound of Formula (1) can be (*R*)-3-amino-4-(5-(bis(2-chloroethyl)amino)-2-methylphenyl)butanoic acid (1b) or a pharmaceutically acceptable zwitterion, internal salt, or salt thereof:

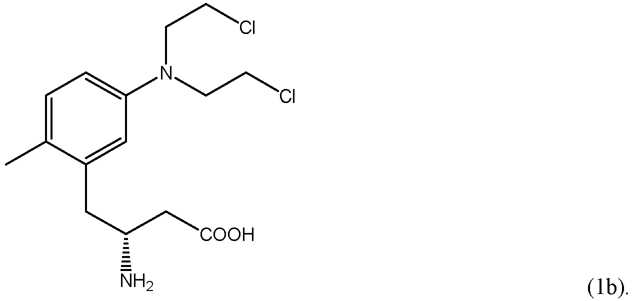

A compound of Formula (1) can be (*S*)-3-amino-4-(5-(bis(2-chloroethyl)amino)-2-methylphenyl)butanoic acid (1c), or a pharmaceutically acceptable zwitterion, internal salt, or salt thereof:

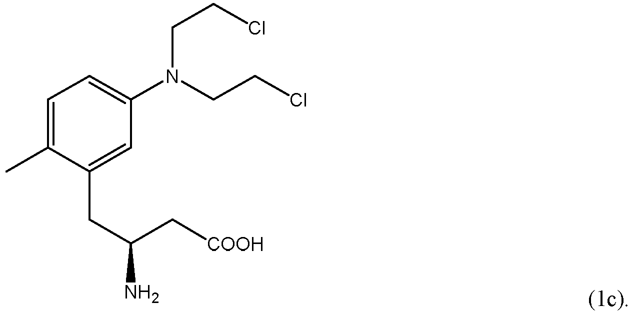

In a compound of Formula (1), the pharmaceutically acceptable salt can be the monohydrochloride salt.

In a compound of Formula (1), the pharmaceutically acceptable salt can be the bis(hydrochloride) salt.

The salt form of a compound of Formula (1) can depend on the pH of the aqueous solution containing the compound of Formula (1).

A compound of Formula (1) can be the free base, the zwitterion, or the internal salt.

A compound of Formula (1) can have an enantiomeric purity, for example, greater than about 90%, greater than about 95%, greater than about 98%, greater than about 99%, greater than about 99.5%, or greater than about 99.9%.

Compounds of Formula (1) are substrates of the LAT1/4F2hc (Large Amino Acid 1 Transporter).

Methods of synthesizing compounds of Formula (1) are disclosed in U.S. Patent No. 9,394,237 and in U.S. Patent No. 9,783,487.

A guest-host inclusion complex or a pharmaceutical composition provided by the present disclosure can comprise a cyclodextrin derivative or a combination of cyclodextrin derivatives.

A cyclodextrin derivative can have the structure of Formula (2): 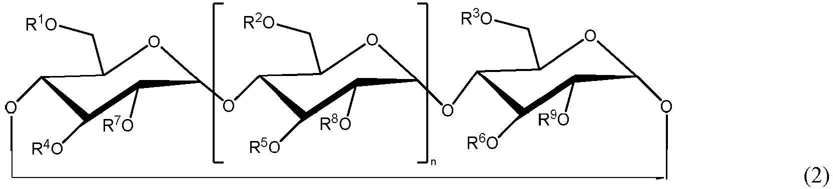 or a pharmaceutically acceptable zwitterion, internal salt, or salt thereof, where,
n can be selected from 4, 5, and 6;
each of R¹ to R⁹ can independently be selected from hydrogen, C₁₋₈ alkanediyl sulfonate salt, C₁₋₆ alkyl, and substituted C₁₋₆ alkyl; and
at least one of R¹ to R⁹ can be C₁₋₈ alkanediyl sulfonate salt.

In a cyclodextrin derivative of Formula (2), n can be 4, 5, or 6.

In a cyclodextrin derivative of Formula (2), a C₁₋₈ alkanediyl sulfonate salt can be selected from, for example, a sulfonate salt of sulfoethyl, sulfopropyl, 1-methyl-sulfopropyl, sulfobutyl, 1-methyl-sulfobutyl, 2-methyl-sulfobutyl, 1-methyl-sulfobut-3-yl, 2-ethyl-sulfobutyl, 3-ethyl-sulfobutyl, sulfopentyl, 1-sulfopent-3-yl, sulfohexyl, sulfoheptyl, and sulfooctyl.

In a cyclodextrin derivative of Formula (2), a C₁₋₈ alkanediyl sulfonate salt can be selected from, for example, -(CH₂)₁-SO₃⁻X⁺, -(CH₂)₂-SO₃⁻X⁺, -(CH₂)₃-SO₃⁻X⁺, -(CH₂)₄-SO₃⁻X⁺, -(CH₂)₅-SO₃⁻X⁺, - (CH₂)₆-SO₃⁻X⁺, -(CH₂)₇-SO₃⁻X⁺, and -(CH₂)₈-SO₃⁻X⁺, where X⁺ can be selected from, for example, Li⁺, Na⁺, K⁺, Mg²⁺, and Ca²⁺.

A C₁₋₆ alkyl group can be selected, for example, from methyl, ethyl, n-propyl, isopropyl, n-butyl, iso-butyl, sec-butyl, *tert-*butyl, n-pentyl, and n-hexyl.

A substituted C₁₋₆ alkyl group can be selected, for example, from substituted methyl, substituted ethyl, substituted n-propyl, substituted isopropyl, substituted n-butyl, substituted iso-butyl, substituted *tert-*butyl, substituted n-pentyl, and substituted n-hexyl.

A substituted C₁₋₆ alkyl group can be a hydroxyl-substituted C₁₋₆ alkyl group such as 2-hydroxypropyl, 3-hydroxypropyl, 2,3-dihydroxypropyl, 3-oxobutyl, or 2-ethoxy-ethyl.

A cyclodextrin derivative of Formula (2) can have an average degree of substitution (ADS), for example, from about 4 to about 8, from about 5 to about 8, from about 5.5 to about 7.5, from about 6 to about 7.5, from about 6 to about 7, or from about 6.5 to about 7.

In a cyclodextrin derivative of Formula (2), each R¹ to R⁹ can independently be selected from hydrogen and a C₁₋₈ alkanediyl sulfonate salt, and can have an ADS, for example, from about 4 to about 8, from about 5 to about 8, from about 5.5 to about 7.5, from about 6 to about 7.5, from about 6 to about 7, or from about 6.5 to about 7.

In a cyclodextrin derivative of Formula (2), at least one of R¹ to R⁹ can be a hydroxy-substituted C₁₋₆ alkyl group such as a hydroxy-substituted C₃ alkyl, and can have an ADS, for example, from about 1 to about 8, from about 2 to about 8, from about 3 to about 7, or from about 4 to about 7.

In a cyclodextrin derivative of Formula (2) the (counter) cation can be selected, for example, from Li⁺, Na⁺, K⁺, Mg²⁺, Ca²⁺, quaternary ammonium cations such as C₁₋₈ tetraalkyl ammonium, and amine cations such as a C₁₋₆ alkylamine, a C₄₋₈ cycloalkylamine, C₁₋₆ alkanolamine, and a C₄₋₈ cycloalkylamine.

In a cyclodextrin derivative of Formula (2) the cation can be Na⁺. A cyclodextrin derivative of Formula (2) can be the polysodium salt or a mixture of salts.

In a cyclodextrin derivative of Formula (2), each of the (counter) cations can be, for example, sodium and the number of sodium cations is equivalent to the number of sulfonate groups.

A cyclodextrin derivative of Formula (2) can have the structure of Formula (2a): 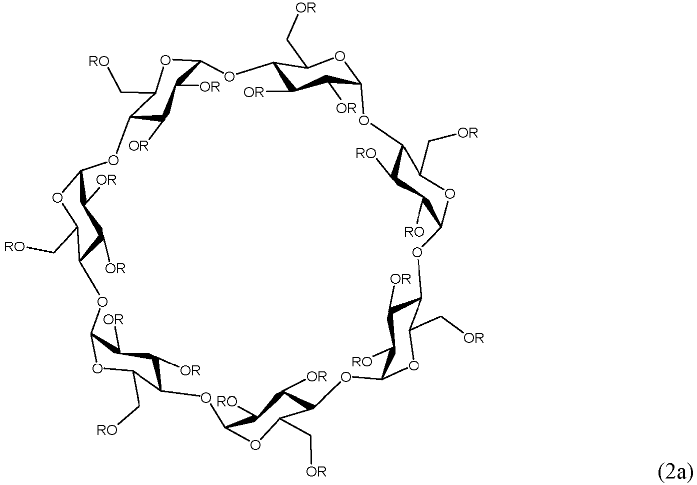 where each R can be independently selected from hydrogen and -(CH₂)₄-SO₃⁻Na⁺.

A cyclodextrin derivative of Formula (2a) can have an ADS, for example, from about 6 to about 7.5, from about 6.2 to about 7.3, or from about 6.5 to about 7.1.

A cyclodextrin derivative of Formula (2a) can be an SBE-β-CD (sulfobutyl ether-β-cyclodextrin) derivative having an ADS, for example, from about 6 to about 7.5, from about 6.2 to about 7.3, or from about 6.5 to about 7.1.

A cyclodextrin derivative of Formula (2a) can be an SBE-β-CD derivative having an ADS, for example, of about 6.0, about 6.1, about 6.2, about 6.3, about 6.4, about 6.5, about 6.6, about 6.7, about 6.8, about 6.9, or about 7.0.

Examples of suitable sulfobutyl ether-β-cyclodextrin derivatives of Formula (2a) having an ADS from about 6 to about 7 are available, for example, as Captisol^{®}. A Captisol^{®} cyclodextrin is a polyanionic β-cyclodextrin derivative with sodium sulfonate salt separated from the lipophilic cyclodextrin cavity by a butyl ether spacer group. Captisol^{®} cyclodextrin derivatives are available from CyDex Pharmaceuticals, Inc, and Ligand Pharmaceuticals. Dexolve^{®} cyclodextrin derivates are also available from CycloLab Cyclodextrin Research and Development Laboratory Ltd.

A cyclodextrin derivative of Formula (2) can comprise SBE_{6.5}-β-CD.

A guest-host inclusion complex provided by the present disclosure can comprise a β-substituted β-amino acid derivative of Formula (1) and a cyclodextrin derivative of Formula (2).

A guest-host inclusion complex can comprise a weight ratio of a compound of Formula (1) to a cyclodextrin of Formula (2), for example, from about 1:1 to about 1:100, from about 1:10 to about 1:90, from about 1:20 to about 1:80, from about 1:30 to about 1:70, or from about 1:40 to about 1:60. A guest-host inclusion complex can comprise a weight ratio of a compound of Formula (1) to a cyclodextrin of Formula (2), for example, from about 1:48 to about 1:60, from about 1:50 to about 1:58, or from about 1:52 to about 1:56.

A guest-host inclusion complex can comprise a molar ratio of a compound of Formula (1) to a cyclodextrin derivative of Formula (2), for example, from about 1:6 to about 1:11, from about 1:7 to about 1:10, from about 1:7.5 to about 1:9.5, from about 1:8 to about 1:9, or from about 1:8.2 to about 1.8.8.

A guest-host inclusion complex can comprise a molar ratio of a compound of Formula (1) to a cyclodextrin derivative of Formula (2), for example, from about 1:7.4 to about 1:9.25.

A guest-host inclusion complex can be a lyophilizate. A lyophilizate can be prepared by dissolving a β-substituted β-amino acid derivative of Formula (1) and a cyclodextrin derivative of Formula (2) in water adjusted to a suitable pH and lyophilizing the solution to provide the corresponding lyophilized guest-host inclusion complex.

A pharmaceutical composition provided by the present disclosure can comprise a guest-host inclusion complex of a β-substituted β-amino acid derivative of Formula (1) and a cyclodextrin derivative of Formula (2).

A pharmaceutical composition provided by the present disclosure can comprise a β-substituted β-amino acid derivative of Formula (1) and a cyclodextrin derivative of Formula (2).

A pharmaceutical composition provided by the present disclosure can comprise a mass ratio of a compound of Formula (1) to Formula (2), for example, from about 1:1 to about 1:100, from about 1:10 to about 1:90, from about 1:20 to about 1:80, from about 1:30 to about 1:70, or from about 1:40 to about 1:60. A pharmaceutical composition can comprise a weight ratio of a compound of Formula (1) to Formula (2), for example, from about 1:48 to about 1:60, from about 1:50 to about 1:58, or from about 1:52 to about 1:56. A 1: 1 mass ratio means 50 mg Compound (1):50 mg cyclodextrin derivative. At an average MW of 2,163 g/mol for SBE_{6.5}-β-CD, this equates to a molar ratio of 150 µmol Compound (1) : 23.1 µmol of SBE_{6.5}-β-CD. A 1:1 molar ratio equates to a mass ratio of 50 mg Compound (1) : 324.5 mg SBE_{6.5}-β-CD or a mass ratio of 1:6.49.

A pharmaceutical composition provided by the present disclosure can comprise a molar ratio of a compound of Formula (1) to Formula (2), for example, from about 1:6 to about 1:11, from about 1:7 to about 1:10, from about 1:7.5 to about 1:9.5, from about 1:8 to about 1:9, or from about 1:8.2 to about 1:8.8.

A pharmaceutical composition can comprise a molar ratio of a compound of Formula (1) to a cyclodextrin derivative of Formula (2), for example, from about 1:7.4 to about 1:9.25.

A pharmaceutical composition can comprise an aqueous formulation.

A reconstituted aqueous formulation can have a nominal concentration of a β-substituted β-amino acid derivative of Formula (1), for example, from about 1 mg/mL to about 9 mg/mL, from about 2 mg/mL to about 8 mg/mL, from about 3 mg/mL to about 7 mg/mL, from about 4 mg/mL to about 6 mg/mL, or from about 4.5 mg/mL to about 5.5 mg/mL.

An aqueous dosing formulation can have a nominal concentration of a β-substituted β-amino acid derivative of Formula (1), for example, from about 0.1 mg/mL to about 2.0 mg/mL, from about 0.2 mg/mL to about 1 mg/mL, from about 0.2 mg/mL to about 0.8 mg/mL, from about 0.3 mg/mL to about 0.7 mg/mL, or from about 0.4 mg/mL to about 0.6 mg/mL.

An aqueous formulation can comprise a combination of a β-substituted β-amino acid derivative of Formula (1) and a cyclodextrin derivative of Formula (2) suspended in a sodium chloride solution, such as from an about 0.4%w/v to about 1.5%w/v sodium chloride solution, from an about 0.6%w/v to about 1.3%w/v sodium chloride solution, or from an about 0.8%w/v to about 1.1%w/v sodium chloride solution. The aqueous solution can be a sodium chloride solution such as about 0.9%w/v normal saline solution.

A pharmaceutical composition provided by the present disclosure can comprise an aqueous solution having a nominal concentration of a compound of Formula (1), for example, from about 3.0 mg/mL to about 7.0 mg/mL such as from about 4.0 mg/mL to about 6.0 mg/mL, or from about 4.5 mg/mL to about 5.5 mg/mL. A pharmaceutical composition can comprise an aqueous solution having a nominal concentration of about 5.0 mg/mL of a compound of Formula (1). The aqueous solution can be a sodium chloride solution such as an about 0.9% w/v normal saline solution, such as about 154 mM sodium chloride solution.

For intravenous administration a pharmaceutical composition can comprise, for example, an aqueous solution having a nominal concentration of a compound of Formula (1) from about 0.3 mg/mL to about 0.7 mg/mL such as from about 0.4 mg/mL to about 0.6 mg/mL, or from about 0.45 mg/mL to about 0.55 mg/mL. A pharmaceutical composition can comprise an aqueous solution having a nominal concentration of about 0.5 mg/mL of a compound of Formula (1). The aqueous solution can be a sodium chloride solution such as an about 0.9% saline solution.

An aqueous formulation can have a pH, for example, from about 3 to about 7, from about 3.5 to about 6.5, from about 4 to about 6, or from about 4 to about 5.

A pharmaceutical composition can comprise a lyophilizate. A lyophilizate can comprise, for example, a lyophilizate of an aqueous formulation provided by the present disclosure. A lyophilizate can be storage stable and can be included, for example, in a kit containing an aqueous diluent such that the lyophilizate can be reconstituted in the aqueous diluent to provide an aqueous formulation such as an injectable aqueous formulation. For example, a lyophilizate provided by the present disclosure can be stable at 5 °C for 6 months, for 12 months or for 18 months. For example, a lyophilizate provided by the present disclosure can be stable at 25 °C/60%RH for 6 months, for 3 months or form 6 months. For example, a lyophilizate provided by the present disclosure can be photostable as determined using the ICH Photostability test method.

A pharmaceutical composition provided by the present disclosure can comprise a mass ratio of a β-substituted β-amino acid derivative of Formula (1) and a cyclodextrin derivative of Formula (2), for example, from about 1:1 to about 1:100, from about 1:10 to about 1:90, from about 1:20 to about 1:80, from about 1:30 to about 1:70, or from about 1:40 to about 1:60. A pharmaceutical composition provided by the present disclosure can comprise a mass ratio of a β-substituted β-amino acid derivative of Formula (1) and a cyclodextrin derivative of Formula (2), for example, from about 1:48 to about 1:60, from about 1:50 to about 1:58, or from about 1:52 to about 1:56.

A pharmaceutical composition provided by the present disclosure can comprise a mass ratio of a β-substituted β-amino acid derivative of Formula (1) and a cyclodextrin derivative of Formula (2) of about 1:54 such as, for example, from about 1:50 to about 1:58, from about 1:51 to about 1:57, from about 1:52 to about 1:56, or from about 1:53 to about 1:55.

A pharmaceutical composition provided by the present disclosure can comprise a molar ratio of a β-substituted β-amino acid derivative of Formula (1) and a cyclodextrin derivative of Formula (2), for example, from about 1:6 to about 1:11, from about 1:7 to about 1:10, from about 1:7.5 to about 1:9.5, from about 1:8 to about 1:9, or from about 1:8.2 to about 1.8.8.

A pharmaceutical composition provided by the present disclosure can comprise, for example, a nominal concentration of a compound of Formula (1) from about 0.25 mg/mL to about 2.0 mg/mL such as about 0.25 mg/mL, about 0.5 mg/mL, about 1.0 mg/mL, about 1.5 mg/mL, or about 2.0 mg/mL.

A pharmaceutical composition provided by the present disclosure can comprise, for example, a sodium chloride solution, such as an about 0.9% w/v saline solution.

A pharmaceutical composition can comprise one or more pharmaceutically acceptable excipients. A pharmaceutical composition can comprise a pharmaceutically acceptable buffer and/or pH adjusting agent such as an acidifying agent or an alkalinizing agent. A pharmaceutical composition can have a pH, for example, from about 3 to about 6, such as from about 4 to about 6, or from about 4.5 to about 5.5, after dilution with an aqueous diluent.

A pharmaceutical composition can comprise a pH-adjusting agent in an amount sufficient to provide a dilute composition having a pH from about 4 to about 6. A pharmaceutical composition can comprise an aqueous NaHCO₃ solution or an aqueous NaOH solution as a pH-adjusting agent. A pharmaceutical composition can comprise aqueous HCl as a pH-adjusting agent.

A pharmaceutical composition provided by the present disclosure such as an aqueous sodium chloride solution can be stable, for example, for about 2 hours, for about 4 hours, or for about 8 hours at a temperature of about 25 °C.

A pharmaceutical composition provided by the present disclosure such as an aqueous sodium chloride solution can be stable, for example, for about 12 hours, for about 24 hours, or for about 36 hours at a temperature of about 0 °C.

A stable pharmaceutical composition refers to a composition in which greater than about 80%, greater than about 85%, greater than about 90%, greater than about 95%, or greater than about 98% of an initial amount of a compound of Formula (1) or a pharmaceutically acceptable zwitterion, internal salt, or salt thereof remains after storage at the stated storage conditions including, for example, the indicated amount of time and at the indicated temperature.

For example, about 0.9% saline solution comprising a concentration of a compound Formula (1)/cyclodextrin guest-host inclusion complex such as a compound of Formula (1c) HCl salt/SBE_{6.5}-β-CD guest-host inclusion complex at a concentration from about 0.125 mg/mL to about 2 mg/mL can be storage stable at about 25 °C for from about 2 to about 12 hours, and at about 0 °C for from about 12 hours to about 48 hours. For example, an about 0.9% saline solution comprising a concentration of a compound Formula (1)/cyclodextrin guest-host inclusion complex such as a compound of Formula (1c) HCl salt/SBE_{6.5}-β-CD guest-host inclusion complex at a concentration from about 0.125 mg/mL to about 2 mg/mL can be storage stable at about 25 °C for about 2 hours, and at about 0 °C for from about 12 hours to about 48 hours.

For example, an about 0.9% saline solution comprising a concentration of a compound Formula (1)/cyclodextrin guest-host inclusion complex such as a compound of Formula (1c) (Compound (1c)) free base/SBE_{6.5}-β-CD guest-host inclusion complex at a concentration from about 0.5 mg/mL to about 2 mg/mL can be storage stable at about 25 °C for from about 5 to about 19 hours, and at about 0 °C for from about 31 hours to about 46 hours.

After a lyophilizate comprising a compound of Formula (1) and a cyclodextrin derivative of Formula (2) can be reconstituted in an aqueous solution such as an about 0.9% saline solution the aqueous pharmaceutical solution can be suitable for intravenous administration, for example, for about 60 minutes, about 90 minutes, for about 120 minutes, for about 180 minutes, or for about 240 minutes.

A pharmaceutical kit provided by the present disclosure can comprise a lyophilizate of a pharmaceutical composition provided by the present disclosure and an aqueous solution for reconstituting the lyophilizate to provide a formulation suitable for intravenous administration. A kit can be used to treat cancer in a patient such as a cancer of the central nervous system, a brain cancer, a metastatic cancer, a metastatic cancer in the central nervous system, or a metastatic cancer in the brain.

A lyophilizate containing a β-substituted β-amino acid derivative of Formula (1) and a cyclodextrin derivative of Formula (2) can be provided in a glass vial fitted with a butyl rubber stopper. For example, the lyophilizate can contain about 50 mg of a β-substituted β-amino acid derivative of Formula (1) and about 2,700 mg of a cyclodextrin derivative of Formula (2).

A kit can optionally comprise an aqueous solution such as an about 0.9% saline solution such as from about 8.0 mL to about 9.0 mL, from about 8.2 mL to about 8.6 mL, or from about 8.3 mL to about 8.5 mL of the 0.9% saline solution. An aqueous solution such as an about 0.9% saline solution can be added to a vial containing the lyophilizate of a compound Formula (1)/cyclodextrin guest-host inclusion complex After mixing the lyophilizate and the saline solution the vial can contain about 10 mL of an aqueous solution having a nominal concentration of a compound of Formula (1) of about 5 mg/mL such as, for example, from about 3 mg/mL to about 7 mg/mL, from about 3.5 mg/mL to about 6.5 mg/mL, from about 4 mg/mL to about 6 mg/mL, or from about 4.5 mg/mL to about 5.5 mg/mL.

A kit can comprise, for example, from about 1 mg to about 100 mg of a lyophilized guest-host inclusion complex, from about 5 mg to about 80 mg, from about 10 mg to about 70 mg, or from about 20 mg to about 60 mg of a lyophilized guest-host inclusion complex.

Any suitable vial size can be used such as from about 10 mL to about 50 mL, from about 10 mL to about 40 mL, or from about 20 mL to about 30 mL. A vial size can be, for example, about 10 mL, about 20 mL, about 30 mL, about 40 mL, or about 50 mL.

This solution can be further diluted by either addition of additional 0.9% saline solution or by diluting an aliquot in another vial or suitable container such as a bag with an appropriate amount of an about 0.9% saline solution to bring the final theoretical or nominal concentration of the compound of Formula (1) to from about 0.2 mg/mL to about 1.0 mg/mL, from about 0.2 mg/mL to about 0.8 mg/mL, from about 0.4 mg/mL to about 0.6 mg/mL, or about 0.5 mg/mL.

The pH of the reconstituted lyophilizate solution can range, for example, from about 4 to about 6, from about 3 to about 5, from about 3.5 to about 4.5, or the pH can be about 4.0.

The reconstituted lyophilizate solution can contain the compound of Formula (1) as the free base, the HCl salt, or a combination thereof. For example, the reconstituted lyophilizate solution of Formula (1) can contain greater than about 0% of the HCl salt, greater than about 20%, greater than about 40%, greater than about 60%, or greater than about 80%, or about 100% of the HCl salt of a compound of Formula (1), where percent is based on the relative molar amount of the compound of Formula (1). For example, about 50 mg of a compound of Formula (1c) corresponds to 0.150038 mmol). For example, a reconstituted solution of Formula (1) can contain from about 0% to about 100% of the HCl salt of a compound of Formula (1), from about 10% to about 90%, from about 20% to about 80%, from about 40% to about 60%, or from about 30% to about 70% of the HCl salt of a compound of Formula (1), where percent is based on the relative molar amount of the compound of Formula (1).

Methods provided by the provided by the present disclosure include methods of administering a pharmaceutical composition provided by the present disclosure to a patient.

A pharmaceutical composition can be administered intravenously such as, for example, by bolus injection, intravenous infusion, limb perfusion, normothermic isolated limb perfusion, percutaneous hepatic perfusion. Intravenous administration includes administration by injection and/or by drip line using a cannula, a central line, a peripherally inserted central catheter line.

A pharmaceutical composition can be administered as an infusion for an appropriate duration.

Pharmaceutical compositions provided by the present disclosure are useful in the treatment of cancer including the treatment of solid tumors and metastases.

The amount of a compound of Formula (1) that will be effective in the treatment of a cancer will depend, at least in part, on the nature of the disease, and may be determined by standard clinical techniques known in the art. In addition, *in vitro* or *in vivo* assays may be employed to help identify optimal dosing ranges. Dosing regimens and dosing intervals may also be determined by methods known to those skilled in the art. The amount of a β-substituted β-amino acid derivative/cyclodextrin derivative administered may depend on, among other factors, the patient being treated, the weight of the patient, potential co-morbidities, the clinical status of the patient, the severity of the disease, the route of administration, and the judgment of the prescribing physician.

For systemic administration, a therapeutically effective dose may be estimated initially from *in vitro* assays. Initial doses may also be estimated from *in vivo* data, *e.g.*, animal models, using techniques that are known in the art. Such information may be used to more accurately determine useful doses in humans. One having ordinary skill in the art may optimize administration to humans based on animal data.

A pharmaceutical composition provided by the present disclosure can be administered, for example, once per day, twice per day, and in certain embodiments at intervals of more than once per day. Dosing may be provided alone or in combination with other drugs and may continue as long as required for effective treatment of the disease. Dosing may also be undertaken using continuous or semi-continuous administration over a period of time. Dosing includes administering a pharmaceutical composition to a mammal, such as a human, in a fed or fasted state.

A dose of a compound of Formula (1) and appropriate dosing intervals may be selected to maintain a sustained therapeutically effective concentration of the compound of Formula (1) in the blood of a patient.

A pharmaceutical composition provided by the present disclosure can be administered using a suitable dosing regimen. A dosing regimen can be adjusted, ceased, or extended to accommodate as appropriate to accomplish the therapeutic objectives.

A pharmaceutical composition comprising a compound of Formula (1) can be administered to treat cancer in a patient so as to provide a therapeutically effective concentration of a compound of Formula (1) in the plasma of the patient. A therapeutically effective concentration of a compound of Formula (1) in the plasma of a patient can be less than an amount that causes unacceptable adverse effects including adverse effects to homeostasis. A therapeutically effective concentration of a compound of Formula (1) in the plasma of a patient is an amount sufficient to treat the cancer in a patient.

A pharmaceutical composition provided by the present disclosure can be administered to treat cancer in a patient so as to provide a therapeutically effective concentration of a compound of Formula (1) in the blood or plasma of a patient for an extended period of time such as, for example, for at least about 0.5 hours, for at least about 1 hour, for at least about 2 hours, for at least about 3 hours, for at least about 4 hours, for at least about 6 hours, for at least about 8 hours, for at least about 10 hours, or for at least about 12 hours.

The amount of a compound of Formula (1) administered may vary during a treatment regimen.

Methods provided by the present disclosure include methods of treating cancer in a patient comprising administering to a patient in need of such treatment a therapeutically effective amount of a pharmaceutical composition provided by the present disclosure to the patient.

A cancer can be a cancer in the brain or a cancer in the central nervous system independent of the origin of the cancer. A cancer can be a metastatic cancer in the brain or in the central nervous system. A cancer can be a solid tumor originating in the brain or in the central nervous system.

A pharmaceutical composition can be used to treat breast cancer and metastatic breast cancer.

Pharmaceutical compositions provided by the present disclosure can be used to treat central nervous system cancers.

A pharmaceutical composition provided by the present disclosure can be used to treat a brain cancer.

A cancer can be a cancer of the central nervous system or a metastasis of a cancer originating in tissue other than in the central nervous system.

A cancer can be a primary brain cancer or a metastatic brain cancer. A cancer can be a primary cancer of the central nervous system or a metastatic central nervous system cancer.

A pharmaceutical composition provided by the present disclosure can be used to treat a metastatic cancer, such as a metastatic cancer of any origin that highly expresses LAT1/4F2hc.

A pharmaceutical composition provided by the present disclosure can be administered with one or more compounds effective in treating the cancer being treated by the compound of Formula (1) and/or a side effect caused by administering the compound of Formula (1)-(1c).

A pharmaceutical composition provided by the present disclosure can be administered concurrently with the administration of another therapeutic agent, which may be part of the same pharmaceutical composition as, or in a different pharmaceutical composition than that comprising a compound of Formula (1). A compound of Formula (1) can be administered prior or subsequent to administration of another therapeutic agent. The combination therapy can comprise alternating between administering a compound of Formula (1) and a composition comprising another therapeutic agent, *e.g.*, to minimize adverse drug effects associated with a particular drug. When a compound of Formula (1) is administered concurrently with another therapeutic agent that potentially may produce an adverse drug effect including, for example, toxicity, the other therapeutic agent may be administered at a dose that falls below the threshold at which the adverse drug reaction is elicited.

A pharmaceutical composition provided by the present disclosure can be administered in conjunction with an agent known or believed to be effective in treating cancer in a patient.

### EXAMPLES

Embodiments provided by the present disclosure are further illustrated by reference to the following examples, which describe pharmaceutical compositions and properties of pharmaceutical compositions provided by the present disclosure. It will be apparent to those skilled in the art that many modifications, both to materials, and methods, may be practiced without departing from the scope of the disclosure.

### Example 1

### Solubility of Compound (1c) Free Base

Mannitol (500 mg; MW 182.17 g/mol; 2.744 mmol) was added to 10 mg (0.030 mmol) of (S)-3-amino-4-(5-(bis(2-chloroethyl)amino)-2-methylphenyl)butanoic acid (Compound (1c)) (free base; MW 333.25 g/mol) and dissolved in 5 mL, 10 mL, or 15 mL of distilled water by vortexing for about 30 sec to provide a solution having a pH from 3.8 to 4.2.

Compound (1c) did not fully dissolve in 5 mL or 10 mL of the mannitol solution. Compound (1c) fully dissolved in 15 mL of the mannitol solution.

### Example 2

### Solubility of Compound (1c) Free Base

Lactose (500 mg; MW 342.30 g/mol; 1.461 mmol) was added to 10 mg (0.030 mmol) of (S)-3-amino-4-(5-(bis(2-chloroethyl)amino)-2-methylphenyl)butanoic acid (Compound (1c)) (free base) and dissolved in 5 mL, 10 mL, or 15 mL of distilled water by vortexing for about 30 sec to provide a solution having a pH from 3.8 to 4.2.

Compound (1c) did not fully dissolve in 5 mL or 10 mL of the lactose solution. Compound (1c) fully dissolved in 15 mL of the lactose solution.

### Example 3

### Stability of Compound (1c) Free Base

A stability assay was carried out in 96-well microtiter plates. (S)-3-Amino-4-(5-(bis(2-chloroethyl)amino)-2-methylphenyl)butanoic acid (Compound (1c) free base) was incubated at 37 °C in phosphate buffered saline (PBS buffer), pH 7.4. The reaction mixtures (30 µL) contained a final concentration of 1 µM of Compound (1c). The percent Compound (1c) remaining in the mixture was determined using LC/MS/MS.

At each of the sampling time points, 300 µL of quench solution (50 vol-% acetonitrile, 50 vol-% methanol containing 1.3% (v/v) of 6N HCl) with internal standards (bucetin for positive ESI mode; warfarin for negative ESI mode) was transferred to each well. Plates were sealed and centrifuged at 4 °C for 15 min at 4,000 rpm. The supernatant was transferred to new plates for LC/MS/MS analysis.

All samples were analyzed on LC/MS/MS using an AB Sciex API 4000^{®} instrument, coupled to a Shimadzu LC-20AD LC Pump system. Analytical samples were separated using a Waters Atlantis T3 dC18 reverse phase HPLC column (20 mm × 2.1 mm) at a flow rate of 0.4 mL/min. The mobile phases consisted of 0.1% formic acid in water (solvent A) and 0.1% formic acid in 100% acetonitrile (solvent B). Details concerning the eluent gradient used are provided in Table 1. The stability results of test compounds in the PBS buffer are provided in Table 2.

**Table 1. Details of the analytical HPLC gradient.**

| Time (min) | Mobile Phase A (%) | Mobile Phase B (%) |
|---|---|---|
| 0 | 98 | 2 |
| 0.3 | 98 | 2 |
| 1.4 | 2 | 98 |
| 2.0 | 2 | 98 |
| 2.0 | 98 | 2 |
| 3.0 | 98 | 2 |
| 3.0 | stop | stop |

**Table 2. Stability of Compound (1c) Free Base in PBS Buffer.**

| Time (min) | 0 | 15 | 30 | 60 | 120 | 240 |
|---|---|---|---|---|---|---|
| Compound (1c) Remaining (%) | 100 | 78 | 68 | 56 | 30 | 10 |

### Example 4

### Stability of Compound (1c) Free Base

A cyclodextrin derivative (270 mg; SBE_{6.5}-β-CD, Captisol^{®}, average MW 2,163 g/mol) was added to 5.5 mg (0.0165 mmol) of (S)-3-amino-4-(5-(bis(2-chloroethyl)amino)-2-methylphenyl)butanoic acid (Compound (1c)) (free base) in a 12 mL glass vial and dissolved in 3 mL to 4 mL of double-distilled water by vortexing for about 30 sec to provide a clear, colorless solution. The solution was filtered through a 0.45 µm nylon syringe filter into a 12 mL glass vial. The filter was washed twice with double-distilled water (each about 0.5 mL) to provide a clear colorless solution. The filtered solution was frozen at -78 °C (dry ice/acetone bath) and the solvent was lyophilized off at about 100 mTorr for about 16 hours to provide a colorless powdery solid consisting of 5.5 mg (0.0165 mmol of Compound (1c) free base and 270 mg of SBE_{6.5}-β-CD, Captisol^{®}). Several vials with the cyclodextrin derivative/Compound (1c) guest-host inclusion complex were prepared according to this method.

The cyclodextrin derivative/Compound (1c) guest-host inclusion complex was reconstituted by dissolving the guest-host inclusion complex in 0.86 mL of 0.9% saline to provide 1.0 mL of a solution having a nominal concentration of 5.5 mg/mL of the cyclodextrin derivative/Compound (1c) guest-host inclusion complex.

A 1.0 mL aliquot of the reconstituted solution was diluted with 10.0 mL of 0.9% saline, and the pH was adjusted with about 5 µL of a saturated aqueous NaHCO₃ solution to provide 11.0 mL of a clear, colorless solution having a nominal concentration of 0.5 mg/mL Compound (1c) at a pH from 5.8 to 6.1.

A 1.0 mL aliquot of the reconstituted solution was diluted with 4.5 mL of 0.9% saline, and the pH was adjusted with about 5 µL of a saturated aqueous NaHCO₃ solution to provide 5.5 mL of a clear, colorless solution having a nominal concentration of 1.0 mg/mL of Compound (1c) and a pH of 6.1.

A 1.0 mL aliquot of the reconstituted solution was diluted with 1.75 mL of 0.9% saline, and the pH was adjusted with about 10 µL of a saturated aqueous NaHCO₃ solution to provide 2.75 mL of a clear, colorless solution having a nominal concentration of 2.0 mg/mL of Compound (1c) and a pH of 6.1.

The solutions were placed in glass vials and stored at either room temperature (about 25 °C) or refrigerated (about 0 °C), and the amount of Compound (1c) was measured at intervals using analytical high-pressure liquid chromatography coupled to a UV detector (HPLC/UV).

Analytical HPLC/UV was performed using an Agilent 1200 HPLC system equipped with a G1379B degasser, a G1312A Bin Pump, a G1367B Hip-Als, a G1316A TCC, a G1315B DAD, a Phenomenex^{®}, Kinetex 5 µm column, C18 (4.6 × 150 mm) and a Zorbax^{®} Eclipse XDB C18 column (2.1 × 150 mm), and a personal computer for data computation. Gradients of water (solvent A) (Arrowhead, Nestle North America, Inc.) and acetonitrile (MeCN; solvent B) (EMD AX0145-1 or Aldrich Chromasolv^{®} 439134) each containing 0.1 vol-% of trifluoroacetic acid (TFA) (Oakwood Chemical, 001271) were used in the analytical HPLC/UV analyses. The HPLC/UV method employed a gradient from 5 vol-% of Solvent B to 100 vol-% of solvent B in 15 min runtime at a flow rate of 1.0 mL/min and UV detection at λ = 220 nm and λ = 254 nm. Details for the eluent gradient are provided in Table 3. The percent of Compound (1c) remaining was based on the AUC determined at an absorption wavelength λ = 254 nm with linear extrapolation to time zero (set as 100% AUC). The results are provided in Table 4.

**Table 3. Details of the analytical HPLC gradient.**

| Time (min) | Mobile Phase A (%) | Mobile Phase B (%) |
|---|---|---|
| 0 | 95 | 5 |
| 15 | 0 | 100 |
| 16 | 0 | 100 |
| 16.5 | 95 | 5 |
| 16.5 | stop | stop |

**Table 4. Stability of Compound (1c) free base.**

| Nominal Concentration of Compound (1c) (mg/mL) | Temperature 25 °C | | Temperature 0 °C | |
|---|---|---|---|---|
| | Hours | Compound (1c) (% AUC) | Hours | Compound (1c) (% AUC) |
| 0.5 | 8 | 66 | 31 | 82 |
| 1.0 | 5 | 90 | 49 | 97 |
| 2.0 | 19 | 84 | 46 | 89 |

### Example 5

### Stability of Compound (1c) Hydrochloride Salt

A stock solution was prepared by dissolving 68.8 mg (0.206 mmol) of Compound (1c) (free base) in about 20.0 mL of a mixture of acetonitrile/ water (1:1, v/v) with vortexing in a clean graduated glass cylinder (25 mL). A solution of 0.407 mL of 0.5072 M aq. HCl (0.206 mmol, 1.0 eq.; Fluka, 318957) was added with gentle vortexing, and the resulting clear solution was further diluted to 25.0 mL with a mixture of acetonitrile/water (1:1, v/v) with gentle vortexing to afford a stock solution of Compound (1c) as a mono-HCl salt of a concentration 2.75 mg/mL. This solution was filtered through a 0.45 µm nylon syringe filter into a clean 50 mL Erlenmeyer flask. Aliquots of 2.0 mL (2 × 1.0 mL; 2 × 2.75 mg) of the stock solution were rapidly pipetted into clean 12 mL scintillation vials. The aliquots were frozen at -78 °C (dry ice/acetone bath) and lyophilized at about 100 mTorr for about 16 hours to provide test vials each containing 6.1 mg (0.0165 mmol) of Compound (1c) mono-hydrochloride salt (mono-HCl salt) (corresponding to 5.5 mg of Compound (1c)),free base, as a colorless powdery solid.

A cyclodextrin derivative (270 mg; SBE_{6.5}-β-CD, Captisol^{®}, average MW 2,163 g/mol) was added to 6.1 mg (0.0165 mmol) of (S)-3-amino-4-(5-(bis(2-chloroethyl)amino)-2-methylphenyl)butanoic acid mono hydrochloride salt (Compound (1c) mono-HCl salt) in a 12 mL glass vial and dissolved in 3 mL to 4 mL of double-distilled water by vortexing for about 30 sec to provide a clear, colorless solution. The solution was filtered through a 0.45 µm nylon syringe filter into a 12 mL glass vial. The filter was washed twice with double-distilled water (each about 0.5 mL) to provide a clear colorless solution having a pH from 4.4 to 4.7. The filtered solution was frozen at -78 °C (dry ice/acetone bath) and the solvent was lyophilized off at about 100 mTorr for about 16 hours to provide a colorless powdery solid consisting of 6.1 mg (0.0165 mmol) of Compound (1c) mono-HCl salt (corresponding to 5.5 mg of Compound (1c) as free base) and 270 mg of SBE_{6.5}-β-CD, Captisol^{®}). Several vials with the cyclodextrin derivative/ Compound (1c) mono-HCl salt guest-host inclusion complex were prepared according to this method.

The cyclodextrin derivative/Compound (1c) mono-HCl salt guest-host inclusion complex was reconstituted by dissolving the guest-host inclusion complex in 0.86 mL of 0.9% saline to provide 1.0 mL of a solution having a nominal concentration of 6.1 mg/mL of Compound (1c) mono-HCl salt guest-host inclusion complex which equates to a nominal concentration of 5.5 mg/mL of the Compound (1c)) guest-host inclusion complex.

A 0.5 mL aliquot of this solution was diluted with 5.0 mL of 0.9% saline, and the pH adjusted with 5 µL of a saturated aqueous NaHCO₃ solution to provide 5.5 mL of a clear, colorless solution having a nominal concentration of 0.5 mg/mL Compound (1c) compound and a pH from 5.3 to 5.5.

A 1.0 mL aliquot of the reconstituted solution was diluted with 4.5 mL of 0.9% saline, and the pH was adjusted with about 10 µL of a saturated aqueous NaHCO₃ solution to provide 5.5 mL of a clear, colorless solution having a nominal concentration of 1.0 mg/mL of Compound (1c) and a pH of 5-6.

A 1.0 mL aliquot of the reconstituted solution was diluted with 1.75 mL of 0.9% saline, and the pH was adjusted with about 10 µL of a saturated aqueous NaHCO₃ solution to provide 2.75 mL of a clear, colorless solution having a nominal concentration of 2.0 mg/mL of Compound (1c) and a pH of 5-6.

A 0.25 mL aliquot of the reconstituted solution was diluted with 11.75 mL of 0.9% saline, and the pH was adjusted with about 10 µL of a saturated aqueous NaHCO₃ solution to provide 11.0 mL of a clear, colorless solution having a nominal concentration of 0.125 mg/mL of Compound (1c) and a pH of greater than 6.

The solutions were placed in glass vials and stored at either room temperature (about 25 °C) or refrigerated (about 0 °C), and the amount of Compound (1c) was measured at intervals using analytical high pressure liquid chromatography coupled to a UV detector (HPLC/UV) as described in Example 4. The results are provided in Table 5.

**Table 5. Stability of Compound (1c) hydrochloride salt in 0.9% saline.**

| Nominal Concentration of Compound (1c) (mg/mL) | Temperature 25 °C | | Temperature 0 °C | |
|---|---|---|---|---|
| | Hours | Compound (1c) (% AUC) | Hours | Compound (1c) (% AUC) |
| 0.125 | 2 | 98 | 31 | 93 |
| 0.5 | 2 | 97 | 9 | 99 |
| 1.0 | 2 | 91 | 31 | 100 |
| 2.0 | 2 | 99 | 31 | 91 |

### Example 6

### Stability of Compound (1c) in Formulations Containing a Compound (1c): SBE_{6.5}-β-CD Guest-Host Inclusion Complex at Different pH Values

The stability of Compound (1c) in formulations containing a Compound (1c): SBE_{6.5}-β-CD guest-host inclusion complex at different pH was determined by analytical HPLC/UV. The mass ratio of Compound (1c) to SBE_{6.5}-β-CD was either 1:54 or 1:50.

To prepare the formulations, SBE_{6.5}-β-CD was dissolved in water for injection (WFI) by continuous stirring until a clear solution was obtained. The solution was acidified by addition of 1 N HCl. Compound (1c) was dissolved in the acidified aqueous SBE_{6.5}-β-CD solution and water was added at Q.S. to the final volume to prepare a solution with a nominal concentration of 5.0 mg/mL of Compound (1c). The pH was adjusted with 1 N HCl to the values shown in Table 7 which corresponded to the pH of the final formulation after reconstitution with 0.9% saline. The solution was filtered through a 0.22 µm syringe filter followed by lyophilization to dryness affording a colorless powdery solid. The freeze-dried lyophilizate was reconstituted with 0.9% saline to the nominal target concentration of 5.0 mg/mL of Compound (1c).

The total AUC of impurities and the AUC of the impurities at the relative retention times (RRT) at 0.42 and 1.27 were also determined by analytical HPLC/UV. The RRT at 0.42 corresponds to the mono-hydrolysis product of Compound (1c). The identity of the impurity at the RRT of 1.27 has not been determined.

Analytical HPLC/UV was performed on a HPLC chromatograph equipped with a UV detector, a Waters Xbridge^{®} C18 column (4.6 × 150 mm; 5 µm) operated at 30 °C and a personal computer for data computation. Gradients of 0.01 M potassium dihydrogen phosphate solution (KH₂PO₄) solution adjusted to pH to 3.0 with phosphoric acid (H₃PO₄):acetonitrile = 850:150 (v/v) (solvent A) and acetonitrile (MeCN; solvent B) were used in the analytical HPLC/UV analyses. The needle wash was performed with 50% aqueous acetonitrile. Prior to injection into the HPLC/UV system (10 µL injection volume), samples were diluted to a nominal concentration of 0.5 mg/mL of Compound (1c) with a 0.9 M sodium chloride (NaCl) and 0.1 N hydrochloric acid (HCl) diluent. The diluent solution was prepared from 5.26 g (90.0 mmol) of sodium chloride and 850 µL of concentrated hydrochloric acid (HCl) (36-38 wt-%, d 1.184, 1.00 g, 10.2 mmol) diluted to 100 mL solution.

The HPLC/UV method used employed a gradient from 10 vol-% of solvent B to 100 vol-% of solvent B with a 36 min runtime at a flow rate of 1.5 mL/min and UV detection at λ = 220 nm. Details of the eluent gradient used are provided in Table 6. The results of the HPLC/UV analysis are provided in Table 7.

**Table 6. Details of the analytical HPLC gradient.**

| Time (min) | Mobile Phase A (%) | Mobile Phase B (%) |
|---|---|---|
| 0 | 90 | 10 |
| 2 | 90 | 10 |
| 15 | 65 | 35 |
| 20 | 50 | 50 |
| 30 | 50 | 50 |
| 30.5 | 90 | 10 |
| 36 | 90 | 10 |
| 36 | stop | stop |

**Table 7. Stability of Compound (1c) in formulations containing a Compound (1c):SBE_{6.5}-β-CD Guest-Host Inclusion Complex in 0.9% saline at different pH values and a nominal concentration of 5.0 mg/mL.**

| Compound (1c) | Free Base | | | Free Base | | | Free Base | | | HCl salt | | |
|---|---|---|---|---|---|---|---|---|---|---|---|---|
| Compound (1c): SBE_{6.5}-β-CD Ratio (% AUC) | 1:54 | | | 1:50 | | | 1:54 | | | 1:54 | | |
| pH | 3 | | | 3.6 | | | 4.1 | | | 4.1 | | |
| Impurity (% AUC) | Total | 0.40 | 1.31 | Total | 0.42 | 1.27 | Total | 0.36 | 1.31 | Total | 0.36 | 1.31 |
| Cake (%) | 1.16 | 0.62 | 0.14 | 2.86 | 2.26 | 0.35 | 2.04 | 1.46 | 0.23 | 2.13 | 1.51 | 0.18 |
| 0 h (%) | 1.14 | 0.64 | 0.13 | 2.92 | 2.28 | 0.36 | 2.01 | 1.46 | 0.19 | 2.2 | 1.51 | 0.18 |

### Example 7

### Stability of Compound (1c) in Formulations Containing Different Compound (1c):SBE_{6.5}-β-CD Guest-Host Inclusion Complex

The stability of Compound (1c) in formulations containing varying ratios of Compound (1c):SBE_{6.5}-β-CD guest-host inclusion complex at pH 4.5 was determined by analytical HPLC/UV. The mass ratio of Compound (1c) to SBE_{6.5}-β-CD was either 1:54 or 1:70.

The formulations were prepared as described for Example 6 except that solutions with a nominal concentration of 3.3 mg/mL of Compound (1c) were prepared, and that the pH of the solutions was adjusted with 1 N HCl to 4.5. The solutions were filtered through a 0.22 µm syringe filter followed by lyophilization to dryness to afford colorless powdery solids. The freeze-dried lyophilizates were reconstituted with 0.9% saline to the nominal target concentration of 0.5 mg/mL of Compound (1c) and a pH of 4.6 and directly subject to HPLC/UV analysis. Both a nominal target concentration of 0.5 mg/mL of Compound (1c) and a pH of 4.6 represents a useful concentration and pH for intravenous infusion.

As described for Example 6, the total AUC of impurities and the AUC of the impurities at the relative retention times (RRT) at 0.42 and 1.27 were also determined by analytical HPLC. The RRT at 0.42 corresponds to the mono-hydrolysis product of Compound (1c). The identity of the impurity at the RRT of 1.27 has not been determined.

The analytical HPLC/UV instrumentation and the analytical HPLC/UV analysis methods used were the same as those for Example 6. The results of the HPLC/UV analysis are provided in Table 8.

**Table 8. Stability of Compound (1c) in formulations containing varying ratios of compound (1c):SBE_{6.5}-β-CD guest-host inclusion complex.**

| Formulation | Compound (1c):SBE_{6.5}-β-CD | | | Compound (1c):SBE_{6.5}-β-CD | | |
|---|---|---|---|---|---|---|
| | Ratio 1:54 (% AUC) | | | Ratio 1:70 (% AUC) | | |
| Impurity (% AUC) | Total | 0.36 | 1.31 | Total | 0.36 | 1.31 |
| 0 h | 2.80 | 2.07 | 0.37 | 2.46 | 1.81 | 0.29 |
| 1 h | 4.29 | 3.50 | 0.41 | 3.84 | 3.12 | 0.34 |
| 2h | 4.96 | 4.13 | 0.43 | 4.26 | 3.63 | 0.25 |
| 4h | 6.52 | 5.62 | 0.48 | 5.74 | 4.91 | 0.42 |

### Example 8

### Stability of Compound (1c) in Formulations Containing a Compound (1c):SBE_{6.5}-β-CD and Mannitol Guest-Host Inclusion Complex

The stability of a formulation containing a 1:50:30 weight ratio of a Compound (1c):SBE_{6.5}-β-CD:mannitol guest-host inclusion complex was compared to the stability of a formulation containing a 1:50 weight ratio of Compound (1c):SBE_{6.5}-β-CD guest-host inclusion complex

The formulations were prepared as described for Example 6. Solutions with a nominal concentration of 5.0 mg/mL of Compound (1c) were prepared, and the pH of the solutions was adjusted with 1 N HCl to 5.5. The solutions were filtered through a 0.22 µm syringe filter followed by lyophilization to dryness to afford colorless powdery solids. The freeze-dried lyophilizates were reconstituted with 0.9% saline to the nominal target concentration of 5.0 mg/mL of Compound (1c) and a pH of 5.5 and directly analyzed using HPLC/UV after dilution to a nominal concentration of 0.5 mg/mL with the 0.9 M NaCl/0.1 N HCl diluent as described in Example 6.

As described for Example 6 and Example 7, the total AUC of impurities and the AUC of the impurities at the relative retention times (RRT) at 0.42 and 1.27 were also determined by analytical HPLC/UV at 25 °C and at a temperature from 2 °C to 8 °C. The RRT at 0.42 corresponds to the mono-hydrolysis product of Compound (1c). The identity of the impurity at the RRT of 1.27 has not been determined.

The analytical HPLC/UV instrumentation and the analytical HPLC/UV analysis methods used were the same as for Example 6 and Example 7. The results of the HPLC/UV analysis are provided in Table 9.

**Table 9. Stability of Compound (1c) in formulations containing a Compound (1c):SBE_{6.5}-β-CD inclusion complex.**

| | Formulation | Compound (1c):SBE_{6.5}-β-CD:Mannitol Ratio 1:50:30 (wt/wt/wt) | | | Compound (1c):SBE_{6.5}-β-CD Ratio 1:50 (wt/wt) | | |
|---|---|---|---|---|---|---|---|
| Condition | Impurity (% AUC) | Total | 0.42 | 1.27 | Total | 0.42 | 1.27 |
| | Compound (1c) | 1.05 | 0.06 | 0.10 | 1.05 | 0.06 | 0.10 |
| | Cake | 5.36 | 3.83 | 1.06 | 5.67 | 4.11 | 1.12 |
| 25 °C | 0 h | 5.30 | 3.79 | 1.05 | 5.69 | 4.14 | 1.10 |
| | 1 h | 5.53 | 3.94 | 1.07 | 5.93 | 4.30 | 1.13 |
| | 2 h | 5.86 | 4.16 | 1.11 | 6.25 | 4.54 | 1.16 |
| | 4 h | 6.36 | 4.53 | 1.15 | 6.73 | 4.90 | 1.21 |
| | 6 h | 6.88 | 4.89 | 1.20 | 7.21 | 5.22 | 1.25 |
| | 24 h | 10.92 | 7.54 | 1.41 | 11.12 | 7.80 | 1.44 |
| 2 °C - 8 °C | 0 h | 5.41 | 3.84 | 1.05 | 5.74 | 4.18 | 1.11 |
| | 1 h | 5.39 | 3.83 | 1.05 | 5.75 | 4.17 | 1.11 |
| | 2h | 5.47 | 3.89 | 1.07 | 5.80 | 4.19 | 1.11 |
| | 4 h | 5.42 | 3.87 | 1.05 | 5.83 | 4.22 | 1.13 |
| | 6 h | 5.42 | 3.83 | 1.06 | 6.00 | 4.26 | 1.20 |
| | 24 h | 5.70 | 4.04 | 1.09 | 6.08 | 4.35 | 1.44 |

### Example 9

### Comparative Stability of Compound (1c), Melphalan and Bendamustine in Injectable Formulations

The stability of Compound (1c) either as the HCl salt or as the free base was compared to the stability of melphalan HCl and/or bendamustine HCl in various pharmaceutical formulations and at different concentrations and at temperatures of 0 °C or 25 °C.

The stability of the compounds in the pharmaceutical formulations was determined at concentrations within the range from 0.4 mg/mL to 0.5 mg/mL, from 0.9 mg/mL to 1.1 mg/mL, and from 1.8 mg/mL to 2.1 mg/mL.

The amount of the active pharmaceutical ingredient (API) (Compound (1c), melphalan or bendamustine) in the pharmaceutical formulations at different times during storage was determined using HPLC/UV as described in detail for Example 4 and Example 5.

Four pharmaceutical compositions were included for the stability measurements: a Na₃-citrate buffered formulation (Formulation 1), a formulation based on Alkeran^{®} (Formulation 2), a formulation based on Evomela^{®} (Formulation 3), and a formulation based on Treanda^{®} (Formulation 4).

### Formulation 1.

The first formulation included a mixture of 57.5 vol-% 1,2-propylene glycol (1,2-PG), 30 vol-% Kolliphor^{®} HS 15 (Solutol^{®}, Macrogol^{®}, BASF AG/SigmaAldrich), and 12.5 vol-% ethanol (EtOH), and the resulting solution was further diluted and adjusted to pH 6.8 using a pH 8.1-8.3 Na₃-citrate buffer (51 mM Na₃-citrate·2H₂O and 73.6 mM NaCl).

The pH 8.1-8.3 Na₃-citrate buffer was prepared form 1.50 g (5.10 mmol) of Na₃-citrate·2H₂O (Na₃C₆H₅O₇·2H₂O ; MW 294.10 g/mol; J.T. Baker, 3650-01) and 0.43 g (7.36 mmol) of sodium chloride (NaCl; MW 58.44 g/mol; SigmaAldrich, S9888) dissolved to 100 mL with double-distilled water (dd-H₂O) (Arrowhead). The solution was filtered through a 0.45 µm nylon syringe filter, and the pH of the solution was adjusted to 8.1-8.3 with a saturated aqueous solution of sodium hydrogencarbonate (NaHCO₃).

The excipient mixture was prepared from 4.77 g (62.7 mmol; 4.6 mL) 1,2-propylene glycol (1,2-PG; MW 76.09 g/mol; d 1.036; Acros Organics, 220870010), 2.52 g (2.40 mL, d 1.05) of melted (15 s microwaving) Kolliphor^{®} HS 15 (BASF AG/SigmaAldrich, 42966), and 0.798 g (17.32 mmol, 1.0 mL) of ethanol (EtOH, MW 46.06 g/mol, d 0.798, KOPTEC, V1016). The clear viscous mixture was warmed to about 40 °C before use (water bath).

A series of 12 mL glass vials each containing 6.1 mg (0.0318 mmol) of Compound (1c) mono-hydrochloride salt (corresponding to 5.5 mg of Compound (1c) as the free base) were prepared as described in Example 5 through lyophilization of aliquots of an acidified stock solution.

660 µL (6 vol-%) of pre-warmed excipient mixture were added to the Compound (1c) mono-hydrochloride salt in the vial. Compound (1c) mono-hydrochloride salt was dissolved with gentle heating and vortexing. The viscous solution was further diluted with 10,340 µL (94 vol-%) of pH 8.1-8.3 Na₃-citrate buffer to provide 11.0 mL of a clear, colorless formulation with a concentration of 0.5 mg/mL of Compound (1c) as a monohydrochloride salt and a pH of 6-7.

330 µL (6 vol-%) of pre-warmed excipient mixture were added to the Compound (1c) mono-hydrochloride salt in the vial. Compound (1c) mono-hydrochloride salt was dissolved with gentle heating and vortexing. The viscous solution was further diluted with 5,170 µL (94 vol-%) of pH 8.1-8.3 Na₃-citrate buffer to provide 5.5 mL of a clear, colorless formulation with a concentration of 1.0 mg/mL of Compound (1c) as a monohydrochloride salt and a pH of 6-7.

165 µL (6 vol-%) of pre-warmed excipient mixture were added to the Compound (1c) mono-hydrochloride salt in the vial. Compound (1c) mono-hydrochloride salt was dissolved with gentle heating and vortexing. The viscous solution was further diluted with 2,585 µL (94 vol-%) of pH 8.1-8.3 Na₃-citrate buffer to provide 2.75 mL of a clear, colorless formulation with a concentration of 2.0 mg/mL of Compound (1c) as a monohydrochloride salt and a pH of 6-7.

The solutions were placed in glass vials and stored at room temperature (about 25 °C), and the amount of Compound (1c) derived from the mono hydrochloride salt (mono HCl salt) of compound of Formula (1c) was measured at intervals using analytical high pressure liquid chromatography coupled to a UV detector (HPLC/UV) as described in Example 4. The results are provided in Table 10.

### Formulation 2.

Alkeran^{®} is an injectable formulation of melphalan-HCl approved by the FDA. Alkeran^{®} for injection is supplied as a sterile, nonpyrogenic, freeze-dried powder. Each vial contains melphalan hydrochloride equivalent to 50 mg melphalan and 20 mg povidone (polyvinylpyrrolidinone, PVP). The solid powder is reconstrued in a sterile diluent containing 0.2 g of sodium citrate, 6.0 mL of propylene glycol, and 0.52 mL of ethanol (96%) and Water for Injection for a total of 10 mL. This provides a nominal 5 mg/mL solution of melphalan (admixture). The dose to be administered is immediately diluted in 0.9% sodium chloride injection, USP, to a concentration not greater than 0.45 mg/mL. Alkeran^{®} for injection is administered intravenously.

A stock solution was prepared by dissolving 68.8 mg (0.206 mmol) of Compound (1c) (free base) and 25.0 mg of Plasdone^{®} C-12 (Povidone; Ashland, 830796) in about 20.0 mL of a mixture of acetonitrile/water (1:1, v/v) with vortexing in clean graduated glass cylinder (25 mL). A solution of 0.407 mL of 0.5072 M aq. HCl (0.206 mmol, 1.0 eq.; Fluka, 318957) was added with gentle vortexing, and the resulting clear solution was further diluted to 25.0 mL with a mixture of acetonitrile/ water (1:1, v/v) with gentle vortexing to afford a stock solution of Compound (1c) as a mono-HCl salt of a concentration 2.75 mg/mL and a Plasdone^{®} concentration of 1.0 mg/mL. This solution was filtered through a 0.45 µm nylon syringe filter into a clean 50 mL Erlenmeyer flask. Aliquots of 2.0 mL (2 × 1.0 mL; 2 × 2.75 mg) of this stock solution were rapidly pipetted into clean 12 mL scintillation vials. The aliquots were frozen at -78 °C (dry ice/acetone bath) and lyophilized at about 100 mTorr for about 16 hours to provide test vials each containing 6.1 mg (0.0165 mmol) of Compound (1c) mono-hydrochloride salt (mono-HCl salt) (corresponding to 5.5 mg of Compound (1c), free base, as a colorless powdery solid.

A stock solution was prepared by dissolving 62.5 mg (0.205 mmol) of melphalan (free base; SigmaAldrich, M2011) and 25.0 mg of Plasdone^{®} C-12 (Povidone; Ashland, 830796) in about 20.0 mL of a mixture of acetonitrile/water (1:1, v/v) with vortexing in clean graduated glass cylinder (25 mL). A solution of 0.404 mL of 0.5072 M aq. HCl (0.205 mmol, 1.0 eq.; Fluka, 318957) was added with gentle vortexing, and the resulting clear solution was further diluted to 25.0 mL with a mixture of acetonitrile/ water (1:1, v/v) with gentle vortexing to afford a stock solution of melphalan as a mono-HCl salt of a concentration 2.50 mg/mL and a Plasdone^{®} concentration of 1.0 mg/mL. This solution was filtered through a 0.45 µm nylon syringe filter into a clean 50 mL Erlenmeyer flask. Aliquots of 2.0 mL (2 × 1.0 mL; 2 × 2.50 mg) of this stock solution were rapidly pipetted into clean 12 mL scintillation vials. The aliquots were frozen at -78 °C (dry ice/acetone bath) and lyophilized at about 100 mTorr for about 16 hours to provide test vials each containing 5.6 mg (0.0164 mmol) of melphalan mono-hydrochloride salt (mono-HCl salt) (corresponding to 5.0 mg of melphalan as a free base) as a colorless powdery solid.

The Alkeran^{®}-type diluent was prepared from 12.43 g (163.4 mmol; 12.0 mL) of 1,2-propylene glycol (1,2-PG; MW 76.09 g/mol; d 1.036; Acros Organics, 220870010), 0.40 g (1.36 mmol) of Na₃-citrate·2H₂O (Na₃C₆H₅O₇·2H₂O; MW 294.10 g/mol; J.T. Baker, 3650-01) and 0.821 g (17.82 mmol, 1.04 mL) of ethanol (EtOH, MW 46.07 g/mol, d 0.798, KOPTEC, V1016) dissolved to 20.0 mL in a 25.0 mL graduated flask. The solution was filtered through a 0.45 µm nylon syringe filter into a clean 20 mL glass vial.

1.0 mL of the Alkeran^{®}-type diluent was added to the Compound (1c) mono-hydrochloride salt in a vial. Compound (1c) mono-hydrochloride salt was dissolved with gentle shaking and vortexing. The solution was further diluted with 9.0 mL of 0.9% saline to provide 10.0 mL of a clear, colorless formulation with a concentration of 0.55 mg/mL of Compound (1c) as a monohydrochloride salt at a pH of 5.3 to 5.7.

0.5 mL of the Alkeran^{®}-type diluent was added to the Compound (1c) mono-hydrochloride salt in the vial. Compound (1c) mono-hydrochloride salt was dissolved with gentle shaking and vortexing. The solution was further diluted with 4.5 mL of 0.9% saline to provide 5.0 mL of a clear, colorless formulation with a concentration of 1.1 mg/mL of Compound (1c) as a monohydrochloride salt at a pH of 5.3.

0.25 mL of the Alkeran^{®}-type diluent was added to the Compound (1c) mono-hydrochloride salt in the vial. Compound (1c) mono-hydrochloride salt was dissolved with gentle shaking and vortexing. The solution was further diluted with 2.25 mL of 0.9% saline to provide 2.5 mL of a clear, colorless formulation with a concentration of 2.2 mg/mL of Compound (1c) as a monohydrochloride salt at a pH of 5.3.

1.0 mL of the Alkeran^{®}-type diluent was added to the melphalan mono-hydrochloride salt in the vial. Melphalan mono-hydrochloride salt was dissolved with gentle shaking and vortexing. The solution was further diluted with 9.0 mL of 0.9% saline to provide 10.0 mL of a clear, colorless formulation with a concentration of 0.50 mg/mL of melphalan as a monohydrochloride salt at a pH of 5.5-5.8.

0.5 mL of the Alkeran^{®}-type diluent was added to the melphalan mono-hydrochloride salt in the vial. Melphalan mono-hydrochloride salt was dissolved with gentle shaking and vortexing. The solution was further diluted with 4.5 mL of 0.9% saline to provide 5.0 mL of a clear, colorless formulation with a concentration of 1.0 mg/mL of melphalan as a monohydrochloride salt at a pH of 5.3-5.5.

0.25 mL of the Alkeran^{®}-type diluent was added to the melphalan mono-hydrochloride salt in the vial. Melphalan mono-hydrochloride salt was dissolved with gentle shaking and vortexing. The solution was further diluted with 2.25 mL of 0.9% saline to provide 2.5 mL of a clear, colorless formulation with a concentration of 2.0 mg/mL of melphalan as a monohydrochloride salt at a pH of 5.0-5.3.

The solutions were placed in glass vials and stored at either room temperature (about 25 °C) and the amount of Compound (1c) derived from the mono-hydrochloride salt (mono-HCl salt) of compound of Formula (1c) or melphalan derived from the mono-hydrochloride salt (mono-HCl salt) of melphalan was measured at intervals using analytical high pressure liquid chromatography coupled to a UV detector (HPLC/UV) as described in Example 4. The results are provided in Table 10.

### Formulation 3.

Evomela^{®} is an injectable formulation of melphalan hydrochloride, 4-[bis(2-chloroethyl)amino]-L-phenylalanine hydrochloride, approved by the FDA. Evomela^{®} is supplied as a sterile white to off-white lyophilized powder in a single-dose vial for intravenous use. Each vial containing 50 mg melphalan free base equivalent to 56 mg melphalan hydrochloride and 2,700 mg betadex sulfobutyl ether (SBE_{6.5}-β-CD; sulfobutyl ether β-cyclodextrin sodium) sodium, NF. Normal saline solution (0.9% Sodium Chloride Injection, USP) (8.6 mL as directed) is used to reconstitute Evomela^{®} to a total of 10 mL having a 5 mg/mL nominal concentration of melphalan. The required volume of Evomela^{®} needed for a patient dose is withdrawn from the vial(s) and added to the appropriate volume of 0.9% Sodium Chloride Injection, USP, to a final nominal concentration of 0.45 mg/mL of melphalan. This solution is then infused via an injection port or central venous catheter.

A series of 12 mL glass vials each containing 5.5 mg (0.0165 mmol of Compound (1c) free base and 270 mg of SBE_{6.5}-β-CD, Captisol^{®}) were prepared as described in Example 4.

The cyclodextrin derivative/Compound (1c) free base guest-host inclusion complex was reconstituted by dissolving the guest-host inclusion complex in 0.86 mL of 0.9% saline to provide 1.0 mL of a solution having a nominal concentration of 5.5 mg/mL of Compound (1c) free base.

A 1.0 mL aliquot of this solution was diluted with 10.0 mL of 0.9% saline, and the pH adjusted with 5 µL of a saturated aqueous NaHCO₃ solution to provide 11.0 mL of a clear, colorless solution having a nominal concentration of 0.50 mg/mL of Compound (1c) from Compound (1c) free base and a pH from 5.8 to 6.1.

A 1.0 mL aliquot of this solution was diluted with 4.5 mL of 0.9% saline, and the pH adjusted with 5 µL of a saturated aqueous NaHCO₃ solution to provide 5.5 mL of a clear, colorless solution having a nominal concentration of 1.0 mg/mL of Compound (1c) from Compound (1c) free base and a pH of about 6.1.

A 1.0 mL aliquot of this solution was diluted with 1.75 mL of 0.9% saline, and the pH adjusted with 5 µL of a saturated aqueous NaHCO₃ solution to provide 2.75 mL of a clear, colorless solution having a nominal concentration of 2.0 mg/mL of Compound (1c) from Compound (1c) free base and a pH of about 6.1.

A series of 12 mL glass vials each containing 6.1 mg (0.0165 mmol of Compound (1c) mono-hydrochloride salt (corresponding to 5.5 mg of Compound (1c) free base) and 270 mg of SBE_{6.5}-β-CD, Captisol^{®}) were prepared as described in Example 5.

The cyclodextrin derivative/Compound (1c) mono-HCl salt guest-host inclusion complex was reconstituted by dissolving the guest-host inclusion complex in 0.86 mL of 0.9% saline to provide 1.0 mL of a solution having a nominal concentration of 6.1 mg/mL of Compound (1c) mono-HCl salt (corresponding to a nominal concentration of 5.5 mg/mL of the Compound (1c) free base).

A 0.5 mL aliquot of this solution was diluted with 5.0 mL of 0.9% saline, and the pH adjusted with 5 µL of a saturated aqueous NaHCO₃ solution to provide 5.5 mL of a clear, colorless solution having a nominal concentration of 0.50 mg/mL of Compound (1c) from Compound (1c) mono-hydrochloride and a pH from 5.3 to 5.5.

A 1.0 mL aliquot of this solution was diluted with 4.5 mL of 0.9% saline, and the pH adjusted with 10 µL of a saturated aqueous NaHCO₃ solution to provide 5.5 mL of a clear, colorless solution having a nominal concentration of 1.0 mg/mL of Compound (1c) from Compound (1c) mono-hydrochloride and a pH from 5 to 6.

A 0.5 mL aliquot of this solution was diluted with 0.875 mL of 0.9% saline, and the pH adjusted with 10 µL of a saturated aqueous NaHCO₃ solution to provide 1.375 mL of a clear, colorless solution having a nominal concentration of 2.0 mg/mL of Compound (1c) from Compound (1c) mono-hydrochloride salt and a pH from 5 to 6.

A 0.25 mL aliquot of this solution was diluted with 10.75 mL of 0.9% saline, and the pH adjusted with 10 µL of a saturated aqueous NaHCO₃ solution to provide 11.0 mL of a clear, colorless solution having a nominal concentration of 0.125 mg/mL of Compound (1c) from Compound (1c) mono-hydrochloride salt and a pH of greater than 6.

A series of 12 mL glass vials each containing 5.0 mg (0.0164 mmol of melphalan free base and 270 mg of SBE_{6.5}-β-CD, Captisol^{®}) were prepared as described in Example 4.

The cyclodextrin derivative/melphalan free base guest-host inclusion complex was reconstituted by dissolving the guest-host inclusion complex in 0.86 mL of 0.9% saline to provide 1.0 mL of a solution having a nominal concentration of 5.0 mg/mL of melphalan free base.

A 1.0 mL aliquot of this solution was diluted with 10.0 mL of 0.9% saline, and the pH adjusted with 5 µL of a saturated aqueous NaHCO₃ solution to provide 11.0 mL of a clear, colorless solution having a nominal concentration of 0.45 mg/mL of melphalan from melphalan free base and a pH from 5.8 to 6.1.

A series of 12 mL glass vials each containing 5.6 mg (0.0164 mmol of melphalan mono-hydrochloride salt (corresponding to 5.0 mg of melphalan free base) and 270 mg of SBE_{6.5}-β-CD (Captisol^{®}) were prepared as described in Example 5.

The cyclodextrin derivative/melphalan mono-HCl salt guest-host inclusion complex was reconstituted by dissolving the guest-host inclusion complex in 0.86 mL of 0.9% saline to provide 1.0 mL of a solution having a nominal concentration of 5.6 mg/mL of melphalan mono-HCl salt (corresponding to a nominal concentration of 5.0 mg/mL of melphalan free base).

A 0.5 mL aliquot of this solution was diluted with 5.0 mL of 0.9% saline, and the pH adjusted with 7 µL of a saturated aqueous NaHCO₃ solution to provide 5.5 mL of a clear, colorless solution having a nominal concentration of 0.45 mg/mL of melphalan from melphalan mono-hydrochloride and a pH from 5.8 to 6.1.

The solutions were placed in glass vials and stored at either room temperature (about 25 °C) and the amount of Compound (1c) resulting from the mono-hydrochloride salt (mono-HCl salt) of compound of Formula (1c) or melphalan resulting from the mono-hydrochloride salt (mono-HCl salt) of melphalan was measured at intervals using analytical high pressure liquid chromatography coupled to a UV detector (HPLC/UV) as described in Example 4. The results are provided in Table 10.

### Formulation 4.

Treanda^{®} is an injectable formulation of bendamustine hydrochloride, 1*H*-benzimidazole-2-butanoic acid, 5-[bis(2-chloroethyl)amino]-1-methyl-, 4-(5-(bis(2-chloroethyl)amino)-1-methyl-1*H-*benzo[d]imidazol-2-yl)butanoic acid monohydrochloride approved by the FDA. Treanda^{®} for injection is provided in a vial containing either 25 or 100 mg of bendamustine HCl as a white to off-white lyophilized powder. Each vial contains either 25 mg or 100 mg of bendamustine hydrochloride and 42.5 mg or 170 mg of mannitol, USP, which is reconstituted in either 0.9% sodium chloride injection, USP, or 2.5% dextrose/0.45% sodium chloride solution injection, USP to a final concentration of 0.2 mg/mL - 0.6 mg/mL and adjusted to a pH from 2.5 to 3.5.

A series of 12 mL glass vials each containing 12.5 mg (0.0317 mmol) of bendamustine mono-hydrochloride salt (C₁₆H₂₁Cl₂N₃O₂·HCl, 394.72 g/mol; MedKoo, 200470) (corresponding to 11.3 mg of bendamustine as the free base) were prepared through weighing out the commercial compound into the vials. To each of the vials either containing bendamustine mono-hydrochloride salt was added 21.3 mg (0.117 mmol) of D-mannitol (C₆H₁₄O₆, MW 182.17 g/mol; SigmaAldrich, M1902). The mixtures were dissolved in 2.0 mL of double distilled water (dd-H₂O) (Arrowhead), the clear colorless solutions were frozen at -78 °C (dry ice/acetone bath), and the solvents were lyophilized off at about 100 mTorr within about 16 h to afford colorless solids.

A stock solution was prepared by dissolving 85.0 mg (0.255 mmol) of Compound (1c) (free base) and 170.4 mg of D-mannitol (0.935 mmol) (C₆H₁₄O₆, MW 182.17 g/mol; SigmaAldrich, M1902), and 0.503 mL of 0.5072 M aq. HCl (0.255 mmol, 1.0 eq.) (Fluka, 318957) in about 16.0 mL of a mixture of acetonitrile/water (1:1, v/v) with vortexing in clean graduated glass cylinder (25 mL) to afford a stock solution of Compound (1c) as a mono-HCl salt of a concentration of 5.313 mg/mL and a D-mannitol concentration of 10.65 mg/mL. This solution was filtered through a 0.22 µm PTFE syringe filter into a clean 25 mL Erlenmeyer flask. Aliquots of 2.0 mL (2 × 1.0 mL; 2 × 5.313 mg) of this stock solution were rapidly pipetted into clean 12 mL scintillation vials. The aliquots were frozen at -78 °C (dry ice/acetone bath) and lyophilized at about 100 mTorr for about 16 hours to provide test vials each containing 11.8 mg (0.0319 mmol) of Compound (1c) mono-hydrochloride salt (mono-HCl salt) (corresponding to 10.6 mg of Compound (1c) as a free base) and 21.3 mg (0.117 mmol) of D-mannitol as a colorless powdery solid.

The materials in the vials were dissolved 2.5 mL of double-distilled water (dd-H₂O) (Arrowhead) to provide clear colorless, non-viscous stock solutions of a concentration of 4.24 mg/mL of Compound (1c) as the monohydrochloride salt or 4.54 mg/mL of bendamustine as the mono-hydrochloride salt, respectively.

1.0 mL of the stock solution containing 4.24 mg/mL of Compound (1c) as the monohydrochloride salt was diluted with 9.0 mL of 0.9% saline to afford 10.0 mL of a test solution of a concentration of 0.424 mg/mL of Compound (1c) as the monohydrochloride salt and with a pH of about 4. 1.0 mL of the stock solution containing 4.24 mg/mL of Compound (1c) as the monohydrochloride salt was diluted with 4.0 mL of 0.9% saline to afford 5.0 mL of a test solution of a concentration of 1.06 mg/mL of Compound (1c) as the monohydrochloride salt and with a pH of 3-4. 1.0 mL of the stock solution containing 4.24 mg/mL of Compound (1c) as the monohydrochloride salt was diluted with 2.0 mL of 0.9% saline to afford 3.0 mL of a test solution of a concentration of 2.12 mg/mL of Compound (1c) as the monohydrochloride salt and with a pH about 3.

1.0 mL of the stock solution containing 4.54 mg/mL of bendamustine as the monohydrochloride salt was diluted with 9.0 mL of 0.9% saline to afford 10.0 mL of a test solution of a concentration of 0.454 mg/mL of bendamustine as the monohydrochloride salt and with a pH of 4-5. 1.0 mL of the stock solution containing 4.54 mg/mL of bendamustine as the monohydrochloride salt was diluted with 4.0 mL of 0.9% saline to afford 5.0 mL of a test solution of a concentration of 1.135 mg/mL of bendamustine as the monohydrochloride salt and with a pH of about 4-4.5. 1.0 mL of the stock solution containing 4.54 mg/mL of bendamustine as the monohydrochloride salt was diluted with 2.0 mL of 0.9% saline to afford 3.0 mL of a test solution of a concentration of 2.27 mg/mL of bendamustine as the monohydrochloride salt and with a pH of about 4.

The solutions were placed in glass vials and stored at either room temperature (about 25 °C) or refrigerated (about 0 °C), and the amount of Compound (1c) resulting from the mono-hydrochloride salt (mono-HCl salt) of compound of Formula (1c) or bendamustine resulting from the mono-hydrochloride salt (mono-HCl salt) was measured at intervals using analytical high pressure liquid chromatography coupled to a UV detector (HPLC/UV) as described in Example 4. The results are provided in Table 10.

**Table 10. Comparative stability of Compound (1c), melphalan, and bendamustine formulations at 25 °C and at 0 °C.**

| Formulation | Temp. | API | Time | Concentration Range | | |
|---|---|---|---|---|---|---|
| | | pH | (hours) | 0.4 - 0.5 mg/mL | 0.9- 1.1 mg/mL | 1.8-2.1 mg/mL |
| | | | | % API ² | | |
| 1 | 25 °C | Compound (1c)·HCl pH 6.8 | 0.5 | 79 | 84 | 88 |
| | | | 2.0 | 52 | 55 | 65 |
| 2 | 25 °C | Melphalan·HCl pH 5.5-5.8 | 0.5 | 95 | 91 | 91 |
| | | | 2.0 | 84 | 71 | 75 |
| | | Compound (1c)·HCl pH 5.5-5.8 | 0.5 | 91 | 89 | 89 |
| | | | 2.0 | 72 | 60 | 62 |
| 3 | 25 °C | Melphalan·HCl pH 5.8-6.1 | 0.5 | 97 | ¹ - | - |
| | | | 2.0 | 92 | | |
| | | Compound (1c)·HCl pH 5-6 | 0.5 | 97 | 97 | 100 |
| | | | 2.0 | 97 | 91 | 99 |
| | 0 °C | Melphalan·HCl pH 5.8-6.1 | 0.5 | 97 | - | - |
| | | | 2.0 | 92 | - | - |
| | | Compound (1c)·HCl pH 5-6 | 0.5 | 97 | 97 | 100 |
| | | | 2.0 | 97 | 91 | 99 |
| | 25 °C | Melphalan free base pH 5.8-6.1 | 0.5 | 97 | ¹- | - |
| | | | 2.0 | 84 | - | - |
| | | Compound (1c) Free Base | 0.5 | 94 | 99 | 100 |
| | | | 2.0 | 84 | 97 | 98 |
| 4 | 25 °C | Bendamustine·HCl pH 4-5 | 0.5 | 98 | 97 | 95 |
| | | | 2.0 | 91 | 95 | 89 |
| | | Compound (1c)·HCl pH 4 | 0.5 | 95 | 95 | 92 |
| | | | 2.0 | 81 | 81 | 75 |
| | 0 °C | Bendamustine·HCl pH 4-5 | 0.5 | 98 | - | - |
| | | | 2.0 | 95 | - | - |
| | | Compound (1c)·HCl pH 4 | 0.5 | 92 | 92 | 93 |
| | | | 2.0 | 78 | 73 | 80 |

| | | | | | | |
|---|---|---|---|---|---|---|
| ¹ Not measured. ² Compound (1c), melphalan or bendamustine. | | | | | | |

Finally, it will be understood that there are alternative ways of implementing the embodiments disclosed herein. Accordingly, the present embodiments are to be considered as illustrative and not restrictive, and the claims are not to be limited to the details provided in the present disclosure.

## Claims

1. A guest-host inclusion complex comprising:
a mass ratio of (*S*)-3-amino-4-(5-(bis-chloroethyl)amino)-2-methylphenyl) butanoic acid (1c) or a pharmaceutically acceptable zwitterion, internal salt, or salt thereof, to a sulfobutyl ether-β-cyclodextrin having an average degree of substitution (ADS) of from 6 to 8 (SBE₆₋₈-β-CD) or a pharmaceutically acceptable zwitterion, internal salt, or salt thereof, of from 1:50 to 1:60.

2. The guest-host inclusion complex according to claim 1, wherein a molar ratio of (S)-3-amino-4-(5-(bis(2-chloroethyl)amino)-2-methylphenyl)butanoic acid (1c), or a pharmaceutically acceptable zwitterion, internal salt, or salt thereof to SBE₆₋₈-β-CD or a pharmaceutically acceptable zwitterion, internal salt, or salt thereof is from 1:7 to 1:10.

3. The guest-host inclusion complex of any one of claims 1 to 2, wherein the guest-host inclusion complex is a lyophilizate.

4. A pharmaceutical composition comprising the guest-host inclusion complex of any one of claims 1 to 3.

5. The pharmaceutical composition of claim 4, wherein the pharmaceutical composition is an aqueous formulation comprising an aqueous diluent.

6. The pharmaceutical composition according to claim 5, wherein the aqueous diluent comprises a sodium chloride solution.

7. The pharmaceutical composition according to claim 5 or 6, wherein the aqueous formulation has a concentration of (*S*)-3-amino-4-(5-(bis(2-chloroethyl)amino)-2-methylphenyl)butanoic acid (1c), or a pharmaceutically acceptable zwitterion, internal salt, or salt thereof of from 0.1 mg/mL to 1.0 mg/mL.

8. The pharmaceutical composition according to any one of claims 5 to 7, wherein the aqueous formulation has a pH from 3 to 7.

9. A pharmaceutical kit comprising: the guest-host inclusion complex of claim 3; and a sodium chloride solution for reconstituting the lyophilizate to provide an aqueous formulation suitable for intravenous administration.

10. The guest-host inclusion complex of any one according to any one of claims 1 to 3, the pharmaceutical composition according to any one of claims 4 to 8, or the kit according to claim 9, for use in a method of treating a brain cancer or metastatic cancer in the brain.

## Patentansprüche

1. Wirt-Gast-Einschlusskomplex umfassend:
ein Masseverhältnis von (S)-3-Amino-4-(5-(bischlorethyl)amino)-2-methylphenyl)butansäure (1c) oder einem pharmazeutisch akzeptablen Zwitterion, internen Salz oder Salz davon zu einem Sulfobutylether-β-cyclodextrin (SBE-₆₋₈-β-CD), das einen durchschnittlichen Substitutionsgrad (ADS) von 6 bis 8 aufweist, oder einem pharmazeutisch akzeptablen Zwitterion, internen Salz oder Salz davon von 1:50 bis 1:60.

2. Wirt-Gast-Einschlusskomplex nach Anspruch 1, wobei ein Molverhältnis von (S)-3-Amino-4-(5-(bischlorethyl)amino)-2-methylphenyl)butansäure (1c) oder einem pharmazeutisch akzeptablen Zwitterion, internen Salz oder Salz davon zu einem SBE-₆₋₈-β-CD oder einem pharmazeutisch akzeptablen Zwitterion, internen Salz oder Salz davon 1:7 bis 1:10 beträgt.

3. Wirt-Gast-Einschlusskomplex nach einem der Ansprüche 1 bis 2, wobei der Wirt-Gast-Einschlusskomplex ein Lyophilisat ist.

4. Pharmazeutische Zusammensetzung umfassend den Wirt-Gast-Einschlusskomplex nach einem der Ansprüche 1 bis 3.

5. Pharmazeutische Zusammensetzung nach Anspruch 4, wobei die pharmazeutische Zusammensetzung eine wässrige Formulierung ist, die ein wässriges Verdünnungsmittel umfasst.

6. Pharmazeutische Zusammensetzung nach Anspruch 5, wobei das wässrige Verdünnungsmittel eine Natriumchloridlösung umfasst.

7. Pharmazeutische Zusammensetzung nach Anspruch 5 oder 6, wobei die wässrige Formulierung eine Konzentration von (S)-3-Amino-4-(5-(bischlorethyl)amino)-2-methylphenyl)butansäure (1c) oder einem pharmazeutisch akzeptablen Zwitterion, internen Salz oder Salz davon von 0,1 mg/ml bis 1,0 mg/ml aufweist.

8. Pharmazeutische Zusammensetzung nach einem der Ansprüche 5 bis 7, wobei die wässrige Formulierung einen pH-Wert von 3 bis 7 aufweist.

9. Pharmazeutisches Kit umfassend: den Wirt-Gast-Einschlusskomplex nach Anspruch 3 und eine Natriumchloridlösung zum Rekonstituieren des Lyophilisats, um eine wässrige Formulierung bereitzustellen, die für intravenöse Verabreichung geeignet ist.

10. Wirt-Gast-Einschlusskomplex nach einem der Ansprüche 1 bis 3, pharmazeutische Zusammensetzung nach einem der Ansprüche 4 bis 8 oder Kit nach Anspruch 9 zur Verwendung bei einem Verfahren zum Behandeln eines Gehirnkrebses oder metastatischen Krebses im Gehirn.

## Revendications

1. Complexe d'inclusion hôte-invité comprenant :
un rapport en masse d'acide (*S*)-3-amino-4-(5-(bis-chloroéthyl)amino)-2-méthylphényl)butanoïque (1c) ou d'un zwitterion, d'un sel interne ou d'un sel pharmaceutiquement acceptables de celui-ci, à une sulfobutyléther-β-cyclodextrine (SBE₆₋₈-β-CD) ayant un degré moyen de substitution (ADS) de 6 à 8 ou à un zwitterion, un sel interne ou un sel pharmaceutiquement acceptables de celle-ci, de 1:50 à 1:60.

2. Complexe d'inclusion hôte-invité selon la revendication 1, dans lequel un rapport molaire d'acide (*S*)-3-amino-4-(5-(bis-chloroéthyl)amino)-2-méthylphényl)butanoïque (1c) ou d'un zwitterion, d'un sel interne ou d'un sel pharmaceutiquement acceptables de celui-ci, à une SBE₆₋₈-β-CD ou à un zwitterion, un sel interne ou un sel pharmaceutiquement acceptables de celle-ci est de 1:7 à 1:10.

3. Complexe d'inclusion hôte-invité selon l'une quelconque des revendications 1 à 2, dans lequel le complexe d'inclusion hôte-invité est un lyophilisat.

4. Composition pharmaceutique comprenant le complexe d'inclusion hôte-invité selon l'une quelconque des revendications 1 à 3.

5. Composition pharmaceutique selon la revendication 4, dans laquelle la composition pharmaceutique est une formulation aqueuse comprenant un diluant aqueux.

6. Composition pharmaceutique selon la revendication 5, dans laquelle le diluant aqueux comprend une solution de chlorure de sodium.

7. Composition pharmaceutique selon la revendication 5 ou 6, dans laquelle la formulation aqueuse a une concentration d'acide (*S*)-3-amino-4-(5-(bis-chloroéthyl)amino)-2-méthylphényl)butanoïque (1c) ou d'un zwitterion, d'un sel interne ou d'un sel pharmaceutiquement acceptables de celui-ci de 0,1 mg/ml à 1,0 mg/ml.

8. Composition pharmaceutique selon l'une quelconque des revendications 5 à 7, dans laquelle la formulation aqueuse a un pH de 3 à 7.

9. Kit pharmaceutique comprenant : le complexe d'inclusion hôte-invité selon la revendication 3, et une solution de chlorure de sodium pour reconstituer le lyophilisat afin de produire une formulation aqueuse adaptée pour une administration intraveineuse.

10. Complexe d'inclusion hôte-invité selon l'une quelconque des revendications 1 à 3, composition pharmaceutique selon l'une quelconque des revendications 4 à 8, ou kit selon la revendication 9, pour utilisation dans une méthode de traitement d'un cancer du cerveau ou d'un cancer métastatique dans le cerveau.
